# EUROPEAN PATENT SPECIFICATION

(11) **EP 3 167 300 B1**
(45) Date of publication and mention of the grant of the patent: **29.09.2021**
(21) Application number: 15818539.7
(22) Date of filing: 09.07.2015
(51) Int. Cl.: G01R 33/00

(54) **METHOD AND SYSTEM FOR SUBSTANCE DETECTION WITH A MAGNETIC SENSOR**
VERFAHREN UND SYSTEM ZUR DETEKTION VON SUBSTANZEN MIT EINEM MAGNETSENSOR
PROCÉDÉ ET SYSTÈME POUR DÉTECTION DE SUBSTANCE À L'AIDE D'UN CAPTEUR MAGNÉTIQUE

(30) Priority: 09.07.2014 US 201461996965 P; 15.06.2015 US 201514738991
(43) Date of publication of application: 17.05.2017
(73) Proprietor: Zepto Life Technology, LLC, St. Paul, Minnesota 55114 (US)
(72) Inventor: QIU, Jiaoming, St. Paul, Minnesota 55114 (US)
(74) Representative: Müller-Boré & Partner Patentanwälte PartG mbB
(86) International application number: PCT/US2015/039747
(87) International publication number: WO 2016/007745

(56) References cited:
- WO-A1-2009/039437
- WO-A2-03/054523
- WO-A2-2007/092909
- US-A- 5 646 001
- US-A- 5 981 297
- US-A1- 2002 119 470
- US-A1- 2002 119 470
- US-A1- 2003 153 092
- US-A1- 2010 324 828
- US-A1- 2011 117 676
- US-A1- 2011 241 664
- US-A1- 2013 102 489
- US-A1- 2014 120 523
- GRAHAM D L ET AL: "Magnetoresistive-based biosensors and biochips", TRENDS IN BIOTECHNOL, ELSEVIER PUBLICATIONS, CAMBRIDGE, GB, vol. 22, no. 9, 1 September 2004 (2004-09-01), pages 455-462, XP004552610, ISSN: 0167-7799, DOI: 10.1016/J.TIBTECH.2004.06.006
- MARTINS V C ET AL: "Femtomolar limit of detection with a magnetoresistive biochip", BIOSENSORS AND BIOELECTRONICS, ELSEVIER BV, NL, vol. 24, no. 8, 15 April 2009 (2009-04-15) , pages 2690-2695, XP026031424, ISSN: 0956-5663, DOI: 10.1016/J.BIOS.2009.01.040 [retrieved on 2009-02-06]
- KOETS M ET AL: "Rapid DNA multi-analyte immunoassay on a magneto-resistance biosensor", BIOSENSORS AND BIOELECTRONICS, ELSEVIER BV, NL, vol. 24, no. 7, 15 March 2009 (2009-03-15) , pages 1893-1898, XP025958950, ISSN: 0956-5663, DOI: 10.1016/J.BIOS.2008.09.023 [retrieved on 2009-02-23]
- XU L ET AL: "Giant magnetoresistive biochip for DNA detection and HPV genotyping", BIOSENSORS AND BIOELECTRONICS, ELSEVIER BV, NL, vol. 24, no. 1, 15 September 2008 (2008-09-15), pages 99-103, XP022820381, ISSN: 0956-5663, DOI: 10.1016/J.BIOS.2008.03.030 [retrieved on 2008-04-08]
- EDELSTEIN R L ET AL: "THE BARC BIOSENSOR APPLIED TO THE DETECTION OF BIOLOGICAL WARFARE AGENTS", BIOSENSORS AND BIOELECTRONICS, ELSEVIER BV, NL, vol. 14, no. 10/11, 1 January 2000 (2000-01-01), pages 805-813, XP001069427, ISSN: 0956-5663, DOI: 10.1016/S0956-5663(99)00054-8
- GRAHAM D L ET AL: "Magnetic field-assisted DNA hybridisation and simultaneous detection using micron-sized spin-valve sensors and magnetic nanoparticles", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, vol. 107, no. 2, 29 June 2005 (2005-06-29) , pages 936-944, XP027810559, ISSN: 0925-4005 [retrieved on 2005-06-29]
- Guichi Zhu ET AL: "Functional nucleic acid-based sensors for heavy metal ion assays", The Analyst, vol. 139, no. 24, 1 January 2014 (2014-01-01), pages 6326-6342, XP055522133, ISSN: 0003-2654, DOI: 10.1039/C4AN01069H

### SEQUENCE LISTING

<110> QIU, JIAOMING
<120> METHOD AND SYSTEM FOR SUBSTANCE DETECTION WITH A MAGNETIC SENSOR
<130> 026462-0431600
<140> TBA
   <141> 2015-07-09
<160> 15
<170> PatentIn version 3.5
<210> 1
   <211> 11
   <212> RNA
   <213> Artificial Sequence
<220>
   <223> Description of Artificial Sequence: Synthetic oligonucleotide
<400> 1
   ccugaggguc g 11
<210> 2
   <211> 33
   <212> DNA
   <213> Artificial Sequence
<220>
   <223> Description of Artificial Sequence: Synthetic oligonucleotide
<400> 2
   catctcttct ccgagccggt cgaaatagtg agt 33
<210> 3
   <211> 33
   <212> DNA
   <213> Artificial Sequence
<220>
   <223> Description of Artificial Sequence: Synthetic oligonucleotide
<220>
   <221> modified_base
   <222> (1)..(9)
   <223> a, c, t, g, unknown or other
<220>
   <221> modified_base
   <222> (25)..(33)
   <223> a, c, t, g, unknown or other
<400> 3
   nnnnnnnnnt ccgagccggt cgaannnnnn nnn 33
<210> 4
   <211> 28
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <221> modified_base
   <222> (1)..(8)
   <223> a, c, t, g, u, unknown or other
<220>
   <221> modified_base
   <222> (21)..(28)
   <223> a, c, t, g, u, unknown or other
<400> 4
   nnnnnnnngu tgaccccuug nnnnnnnn 28
<210> 5
   <211> 21
   <212> DNA
   <213> Artificial Sequence
<220>
   <223> Description of Artificial Sequence: Synthetic oligonucleotide
<220>
   <221> modified_base
   <222> (1)..(5)
   <223> a, c, t, g, unknown or other
<400> 5
   nnnnnctggc cyyyyrrrra c 21
<210> 6
   <211> 47
   <212> DNA
   <213> Artificial Sequence
<220>
   <223> Description of Artificial Sequence: Synthetic oligonucleotide
<400> 6
   cggatagtgt tctttcgcta gaccatgtga cgcatggtga gatgctt 47
<210> 7
   <211> 51
   <212> DNA
   <213> Artificial Sequence
<220>
   <223> Description of Artificial Sequence: Synthetic oligonucleotide
<400> 7
   cacgtccatc tctgcagtcg ggtagttaac cgaccttcag acatagtgag t 51
<210> 8
   <211> 87
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<400> 8
   gcgtgcccgt ctgttgggcc ctaccaatag cacacgugug ugacggccuc gcgcgcccgc 60
   ccugccguag gcacgccccg cgagcuc 87
<210> 9
   <211> 29
   <212> DNA
   <213> Artificial Sequence
<220>
   <223> Description of Artificial Sequence: Synthetic oligonucleotide
<220>
   <221> modified_base
   <222> (1)..(6)
   <223> a, c, t, g, unknown or other
<220>
   <221> modified_base
   <222> (23)..(29)
   <223> a, c, t, g, unknown or other
<400> 9
   nnnnnnrggc tagctacaac gannnnnnn 29
<210> 10
   <211> 13
   <212> DNA
   <213> Artificial Sequence
<220>
   <223> Description of Artificial Sequence: Synthetic oligonucleotide
<400> 10
   gaccgagcca ggc 13
<210> 11
   <211> 20
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<400> 11
   actcactata ggaagagatg 20
<210> 12
   <211> 15
   <212> DNA
   <213> Artificial Sequence
<220>
   <223> Description of Artificial Sequence: Synthetic oligonucleotide
<220>
   <221> modified_base
   <222> (11)..(15)
   <223> a, c, t, g, unknown or other
<400> 12
   yyyyaatacg nnnnn 15
<210> 13
   <211> 13
   <212> DNA
   <213> Artificial Sequence
<220>
   <223> Description of Artificial Sequence: Synthetic oligonucleotide
<400> 13
   ggaacactat ccg 13
<210> 14
   <211> 13
   <212> DNA
   <213> Artificial Sequence
<220>
   <223> Description of Artificial Sequence: Synthetic oligonucleotide
<400> 14
   aagcatctca agc 13
<210> 15
   <211> 26
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<400> 15
   actcactata ggaagagatg gacgtg 26

## Description

The present teaching relates to methods and a medium for metal ion detection. Particularly, the present teaching is directed to methods for detecting a level of a metal ion and a machine-readable tangible and non-transitory medium having information for detecting a level of a metal ion with a magnetic sensor.

The fast development of industry causes more and more pollution in people's living environment. Heavy metals, in form of either compounds or ions, are pollutants gaining more attention recently because they are highly toxic and can have lethal effects on living systems. The detection and monitoring of their existence in water, soil, and living species are desirable and required by laws around the world.

Most of existing technologies to detect heavy metals are expensive, time-consuming, or implemented in bulky devices so that they are difficult to use for field detection. Conventional sensors for detecting metal ions include fluorescent, colorimetric, and, electrochemical sensors, each of which can have a poor performance due to a significant matrix effect, i.e., matrix (all components except the metal ions) in a sample can seriously affect the accuracy of a detection result. Some recent work about metal ion detection using a magnetic sensor merely discloses detecting a metal ion using an additive-signal method, where ion concentration is proportional to signal increase due to some chemical or biochemical reactions. D. L. Graham et al. (Trends in Biotechnol., 2004, 22, 455-462) describe magnetoresistive-based biosensors and biochips. V. C. Martins et al. (Biosensors and Bioelectronics, 2009, 24, 2690-2695) describe the femtomolar limit of detection with a magnetoresistive biochip. M. Koets et al. (Biosensors and Bioelectronics, 2009, 24, 1893-1898) describe a rapid DNA multi-analyte immunoassay on a magneto-resistance biosensor. L. Xu et al. (Biosensors and Bioelectronics, 2008, 24, 99-103) relate to a giant magnetoresistive biochip for DNA detection and HPV genotyping. Patent document WO 2007/092909 A2 describes molecular interaction sensors. Patent document US 2011/241664 A1 concerns a magnetic biosensor and a magnetic biosensor array comprising the same. Patent document WO 2009/039437 A1 describes analyte detection with magnetic sensors. R. L. Edelstein et al. (Biosensors and Bioelectronics, 2000, 14, 805-813) relate to a BARC biosensor applied to the detection of biological warfare agents. D. L. Graham et al. (Sensors and Actuators B: Chemical, 2005, 107, 936-944) describe magnetic field-assisted DNA hybridisation and simultaneous detection using micron-sized spin-valve sensors and magnetic nanoparticles. Patent document WO 03/054523 A2 disclose a sensor and a method for measuring the areal density of magnetic nanoparticles on a micro-array. Patent document US 2002/119470 A1 concerns a magnetic bead-based array for genetic detection. G. Zhu et al. (Analyst 2014, 139, 6326-6342) describe functional nucleic acid-based sensors for heavy metal assay. But the additive-signal method requires a long time for the reactions and hence the ion detection to finish; and is thus not suitable for field use.

Therefore, there is a need to provide an improved solution for metal ion detection using a magnetic sensor to avoid the above-mentioned drawbacks.

The present teaching relates to methods, systems, and programming for metal ion detection. Particularly, the present teaching is directed to methods, systems, and programming for detecting of a level of a metal ion with a magnetic sensor. The present invention is as defined in the appended claims. Embodiments and aspects described herein which are not covered by the claim merely serve to illustrate the technical context of the present invention.

In one example, a method, implemented on a machine having at least one processor and storage, for creating a device is presented. The device may be a signal reading device or a sensing device. A magnetic sensor having one or more layers is formed on a base for sensing a magnetic field created by magnetic particles present in proximity to the magnetic sensor. A first end of each of a first set of breakable strands, wherein said breakable strands comprise functional nucleic acids having metal specificity, is immobilized with respect to the magnetic sensor. A magnetic particle is attached to a second end of each of the first set of breakable strands so that when a material containing a meal ion is in contact with the base, the metal ion causes at least some of the first set of breakable strands to break resulting in that the magnetic particle attached to the second end of each of the at least some of the first set of breakable strands is no longer in proximity to the magnetic sensor.

In another example, a method, implemented on a machine having at least one processor and storage, for detecting a level of a metal ion in a material is presented. A first level of a magnetic field is detected via a device comprising a magnetic sensor. The magnetic sensor is configured for sensing the magnetic field created by magnetic particles present in proximity to the magnetic sensor. The device comprises a first set of breakable strands, wherein said breakable strands comprise functional nucleic acids having metal specificity, each of which having a first end previously immobilized with respect to the magnetic sensor and a second end attached to a magnetic particle. A sample of the material is deposited onto a base of the device that causes, due to the presence of the metal ion, at least some of the first set of breakable strands to break so that the magnetic particle attached to the second end of each of the at least some of the first set of breakable strands is no longer in proximity to the magnetic sensor. A second level of the magnetic field is detected via the device. An amount of the metal ion present in the sample of the material is determined based on the first and second levels of the magnetic field.

In yet another example, a device is presented. The device comprises a base, a magnetic sensor on the base, a first set of breakable strands, wherein said breakable strands comprise functional nucleic acids having metal specificity, and a set of magnetic particles. The magnetic sensor comprises one or more layers for sensing a magnetic field created by magnetic particles present in proximity to the magnetic sensor. Each of the first set of breakable strands has a first end immobilized with respect to the magnetic sensor. Each of the set of magnetic particles is attached to a second end of one of the first set of breakable strands. When a material containing a metal ion is in contact with the base, the metal ion causes at least some of the first set of breakable strands to break so that the magnetic particle attached to the second end of each of the at least some of the first set of breakable strands is no longer in proximity to the magnetic sensor.

In a different example, a system having at least one processor and storage for detecting a level of a metal ion in a material is presented. The system includes a device and a metal ion detector. The device comprises a base, a magnetic sensor on the base, a first set of breakable strands, wherein said breakable strands comprise functional nucleic acids having metal specificity, and a set of magnetic particles. The magnetic sensor is configured for sensing a magnetic field created by magnetic particles present in proximity to the magnetic sensor. Each of the first set of breakable strands has a first end immobilized with respect to the magnetic sensor. Each of the set of magnetic particles is attached to a second end of one of the first set of breakable strands. When a sample of the material is deposited onto the base, the metal ion causes at least some of the first set of breakable strands to break so that the magnetic particle attached to the second end of each of the at least some of the first set of breakable strands is no longer in proximity to the magnetic sensor. The metal ion detector comprises a signal processor and a metal ion level estimator. The signal processor is configured to determine, via the device, a first level of the magnetic field before the sample of the material is deposited and a second level of the magnetic field after the sample of the material is deposited. The metal ion level estimator is configured to determine an amount of the metal ion present in the sample of the material based on the first and second levels of the magnetic field.

Other concepts relate to software for metal ion detection. A software product, in accord with this concept, includes at least one non-transitory machine-readable medium and information carried by the medium. The information carried by the medium may be executable program code data regarding parameters in association with a request or operational parameters, such as information related to a user, a request, or a social group, etc.

In one example, a non-transitory machine readable medium having information recorded thereon for creating a device is presented. The recorded information, when read by the machine, causes the machine to perform the following. A magnetic sensor having one or more layers is formed on a base for sensing a magnetic field created by magnetic particles present in proximity to the magnetic sensor. A first end of each of a first set of breakable strands, wherein said breakable strands comprise functional nucleic acids having metal specificity, is immobilized with respect to the magnetic sensor. A magnetic particle is attached to a second end of each of the first set of breakable strands so that when a material containing a metal ion is in contact with the base, the metal ion causes at least some of the first set of breakable strands to break resulting in that the magnetic particle attached to the second end of each of the at least some of the first set of breakable strands is no longer in proximity to the magnetic sensor.

In another example, a non-transitory machine readable medium having information recorded thereon for detecting a level of a metal ion in a material is presented. The recorded information, when read by the machine, causes the machine to perform the following. A first level of a magnetic field is detected via a device comprising a magnetic sensor. The magnetic sensor is configured for sensing the magnetic field created by magnetic particles present in proximity to the magnetic sensor. The device comprises a first set of breakable strands, wherein said breakable strands comprise functional nucleic acids having metal specificity, each of which having a first end previously immobilized with respect to the magnetic sensor and a second end attached to a magnetic particle. A sample of the material is deposited onto a base of the device that causes, due to the presence of the metal ion, at least some of the first set of breakable strands to break so that the magnetic particle attached to the second end of each of the at least some of the first set of breakable strands is no longer in proximity to the magnetic sensor. A second level of the magnetic field is detected via the device. An amount of the metal ion present in the sample of the material is determined based on the first and second levels of the magnetic field.

### Brief description of the drawings

The methods, systems, and/or programming described herein are further described in terms of exemplary embodiments. These exemplary embodiments are described in detail with reference to the drawings. These embodiments are non-limiting exemplary embodiments, in which like reference numerals represent similar structures throughout the several views of the drawings, and wherein:
FIG. 1 (PRIOR ART) depicts an exemplary structure and characteristic for a giant magnetoresistance (GMR) sensor;
FIG. 2 (PRIOR ART) illustrates an exemplary process for detecting metal ions based on an additive-signal method;
FIG. 3 illustrates an exemplary process for detecting metal ions based on a subtractive-signal method, according to an embodiment of the present teaching;
FIG. 4 illustrates an exemplary process for creating a sensing device for metal ion detection, according to an embodiment of the present teaching;
FIG. 5 is a flow chart of an exemplary process for creating a sensing device for metal ion detection, according to an embodiment of the present teaching;
FIG. 6 illustrates an exemplary diagram of a sensing device generator, according to an embodiment of the present teaching;
FIG. 7 is a flowchart of an exemplary process performed by a sensing device generator, according to an embodiment of the present teaching;
FIG. 8 illustrates an exemplary diagram of a dispensing controller in a sensing device generator, according to an embodiment of the present teaching;
FIG. 9 is a flowchart of an exemplary process performed by a dispensing controller, according to an embodiment of the present teaching;
FIG. 10 illustrates an exemplary diagram of a dispenser in a sensing device generator, according to an embodiment of the present teaching;
FIG. 11 is a flowchart of an exemplary process performed by a dispenser, according to an embodiment of the present teaching;
FIG. 12 illustrates an exemplary process for detecting a metal ion in a sample based on a subtractive-signal method, according to an embodiment of the present teaching;
FIG. 13 illustrates an exemplary diagram of a metal ion detector, according to an embodiment of the present teaching;
FIG. 14 is a flowchart of an exemplary process performed by a metal ion detector, according to an embodiment of the present teaching;
FIG. 15 illustrates an exemplary diagram of a detection controller in a metal ion detector, according to an embodiment of the present teaching;
FIG. 16 is a flowchart of an exemplary process performed by a detection controller, according to an embodiment of the present teaching;
FIG. 17 illustrates an exemplary diagram of a level calculator in a metal ion detector, according to an embodiment of the present teaching;
FIG. 18 is a flowchart of an exemplary process performed by a level calculator, according to an embodiment of the present teaching;
FIG. 19 illustrates an exemplary process for detecting a metal ion with magnetic particles and one set of breakable strands, according to an embodiment of the present teaching;
FIG. 20 illustrates exemplary signal changing processes for a multi-metal ion detection using multiple magnetic sensors on a same device, according to an embodiment of the present teaching;
FIG. 21 illustrates an exemplary process for detecting metal ions based on substrate and catalytic deoxyribonucleic acid (DNA) strands, according to an embodiment of the present teaching;
FIG. 22 shows a table listing examples of metal ions to be detected, where each metal ion can be detected using a corresponding catalytic strand (SEQ ID NOS 1-9, respectively, in order of appearance) and a corresponding substrate strand (SEQ ID NOS 10-15 respectively, in order of appearance) listed in the row of the metal ion, according to different embodiments of the present teaching;
FIG. 23 depicts a general mobile device architecture on which the present teaching can be implemented; and
FIG. 24 depicts a general computer architecture on which the present teaching can be implemented.

In the following detailed description, numerous specific details are set forth by way of examples in order to provide a thorough understanding of the relevant teachings. However, it should be apparent to those skilled in the art that the present teachings may be practiced without such details. In other instances, well known methods, procedures, systems, components, and/or circuitry have been described at a relatively high-level, without detail, in order to avoid unnecessarily obscuring aspects of the present teachings.

The present disclosure describes method, system, and programming aspects of efficient and effective metal ion detection. The method and system as disclosed herein aim at improving a user's metal ion detection experience by providing accurate and prompt metal ion detection.

An antibody-antigen based detection approach has been used to detect an analyte with high selectivity and sensitivity, because of the specific recognition relationship between the antibody and antigen. While heavy metal ions do not have specific recognition to each other, metal ion detection can be performed based on metal specificity of functional nucleic acids (FNA's). This is because FNA's may work like antibodies in recognizing biological species and also have a high level of specificity or selectivity. In addition, FNA's can also work as enzymes to catalyze biochemical reactions in biological groups including nucleic acids, where FNA's are referred as nucleic acid enzymes (NAE's). Most of the NAE's require some specific metal ion to become active and therefore can be used for metal sensing or detection.

According to various embodiments of the present teaching, method and devices have been disclosed to detect heavy metal ions by labeling FNA's with magnetic nanoparticles and sensing a magnetic field created by the magnetic nanoparticles via a magnetic sensor, e.g. a GMR sensor. Because almost all biological specimens and metal ions are nonmagnetic, a detection based on the magnetic sensor is immune to matrix effect in most of the field applications. In addition, an array of magnetic sensors can be formed on a same chip so that multiple ions can be detected with specificity within the same sample at the same time.

Furthermore, metal ion detection in the present teaching may utilize a subtractive-signal method where ion concentration is proportional to signal reduction due to a chemical or biochemical reaction, e.g., a hydrolysis reaction of a DNA strand catalyzed by the metal ion. Compared to an additive-signal method, the subtractive-signal method requires less time to finish the reaction so that it is faster for ion detection and more suitable for field use, when a user does not have much time to wait for a detection result.

Additional novel features will be set forth in part in the description which follows, and in part will become apparent to those skilled in the art upon examination of the following and the accompanying drawings or may be learned by production or operation of the examples. The novel features of the present teachings may be realized and attained by practice or use of various aspects of the methodologies, instrumentalities and combinations set forth in the detailed examples discussed below.

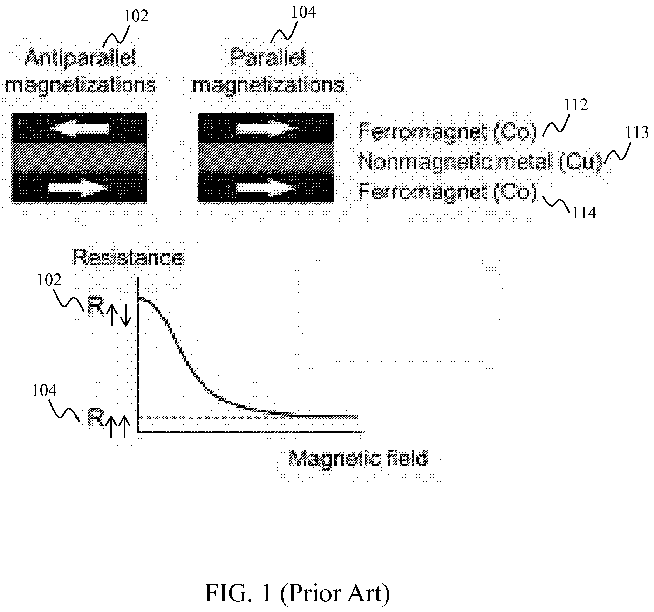

FIG. 1 (PRIOR ART) depicts an exemplary structure and characteristic for a GMR sensor. A GMR sensor is one type of magnetic field sensors that utilize a giant magnetoresistance effect: when there are two magnetic layers separated by one thin nonmagnetic layer, their relative magnetization directions (parallel or antiparallel) result in different resistance states. As shown in FIG. 1, a GMR sensor includes three layers: two ferromagnetic layers 112, 114 comprising Co separated by one nonmagnetic layer 113 comprising Cu. A resistance corresponding to parallel magnetization 104 is smaller than a resistance corresponding to antiparallel magnetization 102.

To utilize the GMR effect, a spin valve structure is usually built by fixing one layer's magnetization and let the other one to rotate freely. The fixed layer is called the reference layer and the rotatable layer is called freelayer. The reference layer may be confined by an antiferromagnetic layer (comprising PtMn or IrMn) directly or indirectly through a so-called SAF (synthetic antiferromagnetic) structure. When the spin valve GMR sensor senses a magnetic field, the freelayer will rotate so that GMR resistance is changed. Typically a static biasing current is applied through the GMR structure so a voltage change is measured instead of the resistance itself.

Preparation of current perpendicular to plane (CPP) GMR sensors may comprise bottom contact deposition, GMR stack deposition, top contact deposition, and sensor structure patterning involving photolithography, iron milling and isolator backfill, etc. Preparation of current in plane (CIP) GMR sensors may comprise GMR stack deposition, top contact deposition, and sensor structure patterning involving photolithography, iron milling and isolator backfill, etc.

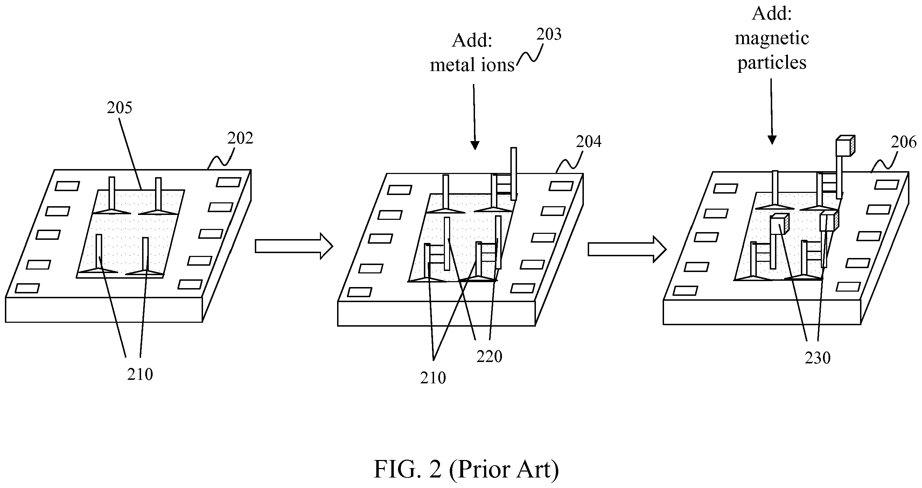

FIG. 2 (PRIOR ART) illustrates an exemplary process for detecting metal ions based on an additive-signal method. At 202, a first set of strands 210 is attached to a magnetic sensor 205 on a chip. The magnetic sensor 205 can sense a magnetic field in proximity to the magnetic sensor 205. At 202, because there is no magnetic material in its proximity, a signal level of magnetic field sensed by the magnetic sensor 205 is zero or a fixed number close to zero.

At 204, a sample of material including a type of metal ions 203 to be detected is added onto the chip. A second set of strands 220 is deposited into the sample on the chip so that at least some of the second set of strands 220 can be bound to the first set of strands 210 due to the presence of the metal ions 203 in the sample.

At 206, magnetic particles 230 are added into the sample on the chip, so that each of the second set of strands 220 bound to the first set of strands 210 is attached to a magnetic particle that becomes in proximity to the magnetic sensor 205 and increases a signal level of the magnetic field in proximity to the magnetic sensor 205. Therefore, an amount of the metal ions 203 in the sample is proportional to an amount of magnetic particles in proximity to the magnetic sensor 205, and is thus proportional to a signal increase of the magnetic field sensed by the magnetic sensor 205. An amount of the metal ions 203 in the sample can be determined based on the sensed signal increase and a predetermined relationship between signal increases and metal ion amounts. Given the amount of the sample added onto the chip, a level of the metal ion in the sample can then be detected based on the determined amount of the metal ions 203 in the sample.

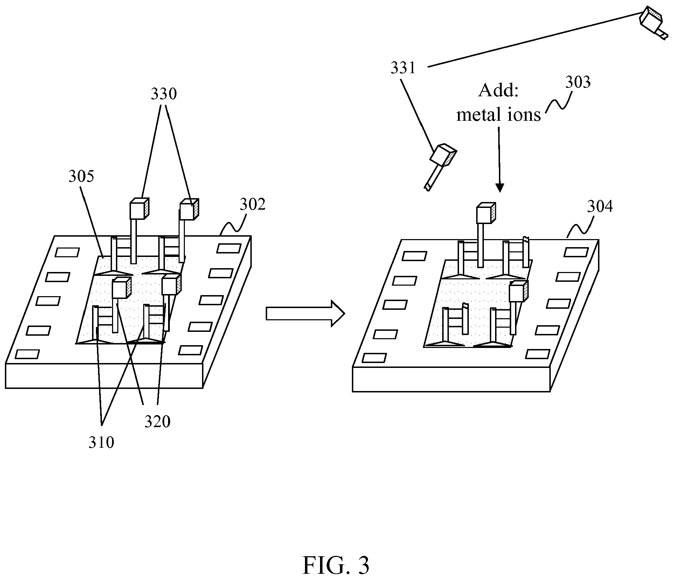

FIG. 3 illustrates an exemplary process for detecting metal ions based on a subtractive-signal method, according to an embodiment of the present teaching. As shown in FIG. 3, at 302, a first set of breakable strands 310, wherein said breakable strands comprise functional nucleic acids having metal specificity, has been attached to a magnetic sensor 305 on a chip. Each of the first set of breakable strands 310 has been bound to one of a second set of strands 320. Each of the second set of strands 320 bound to the first set of breakable strands 310 has been attached to a magnetic particle 330. For example, a first end of each of the second set of strands 320 is bound to one of the first set of breakable strands 310, while a second end of each of the second set of strands 320 is attached to a magnetic particle 330.

Therefore, at 302, there has already been a set of magnetic particles immobilized with respect to the chip and in proximity to the magnetic sensor 305, before any metal ions are added. The magnetic sensor 305 can sense a magnetic field in proximity to the magnetic sensor 305. At 302, because each strand pair 310 and 320 has a corresponding magnetic particle 330 immobilized in proximity to the magnetic sensor 305, a signal level of magnetic field sensed by the magnetic sensor 305 is at a maximum level or a fixed large level.

At 304, a sample of material including a type of metal ions 303 to be detected is added onto the chip, so that at least some of the second set of strands 320 may break due to the presence of the metal ions 303 in the sample. Thus, the magnetic particle 331 attached to each broken strand of the second set of strands is no longer in proximity to the magnetic sensor 305, which causes a reduction of the signal level of the magnetic field sensed by the magnetic sensor 305.

Therefore, an amount of the metal ions 303 in the sample is proportional to an amount of magnetic particles moving out of proximity to the magnetic sensor 305, and is thus proportional to a signal reduction of the magnetic field sensed by the magnetic sensor 305. An amount of the metal ions 303 in the sample can be determined based on the sensed signal reduction and a predetermined relationship between signal reductions and metal ion amounts. Given the amount of the sample added onto the chip, a level of the metal ion in the sample can then be detected based on the determined amount of the metal ions 303 in the sample.

In one embodiment, the first set of breakable strands 310 includes catalytic DNA strands and the second set of strands 320 includes substrate DNA strands. The chip may be a biochip coated with the catalytic DNA strands each of which is bound to a substrate DNA strand. Each substrate DNA strand has a corresponding magnetic particle, e.g. a magnetic nanoparticle (MNP), attached to its end. The substrate DNA strand may or may not be attached to a surface of the chip. In either case, the substrate DNA strand and its corresponding MNP are immobilized with respect to the chip and the magnetic sensor, because the substrate DNA strand is bound to a catalytic DNA strand that is coated on the chip. Once the substrate DNA strand breaks due to the presence of some metal ion, the MNP attached to the end of the substrate DNA strand will become mobilized and flow away from the magnetic sensor, although the MNP was immobilized in proximity to the magnetic sensor before the metal ion was added.

In accordance with various embodiments, the first set of breakable strands 310 may be immobilized on the chip based on a methodology other than coating, e.g., based on covalent linking. The covalent linking may be accomplished via several routes starting from an oxide or nitride passivated chip surface: (1) using Schiff base chemistry with silane based NH₂- containing molecule (APTES or (3-aminopropyl)triethoxysilane, APDES or (3-aminopropyl)(diethoxy)methylsilane, APMES or (3-aminopropyl)(monoethoxy)dimethylsilane) condensing with double-aldehyde linker (Glutaldehyde); (2) silane based NH₂- containing molecules (APTES, APDES, APMES), followed by sulfo-SMCC or (sulfosuccinimidyl 4-(N-maleimidomethyl)cyclohexane-1-carboxylate); or (3) silane based PEG or polyethylene glycol with NHS (N-Hydroxysuccinimide ester)-functional groups.

In accordance with various embodiments, the MNP's can be made with magnetic material such as iron and its alloys, cobalt and its alloys, nickel and its alloys, iron-, cobalt-, or nickel-containing oxides. Typical examples include FeCo, FeNi, FeCoNi, Fe₂O₃, Fe₃O₄, and the combinations of Fe, Co, Ni, O, N, C, Si, or the like. MNP's can be fabricated using various techniques, including both chemical synthesis and physical deposition. The size of MNP can be in the range of, for example, 3 to 500 nm and typically 10 to 80 nm. The size of MNP is may beneficially be comparable to biomolecules (e.g. DNA or RNA) so that MNP can provide high sensitivity. MNP's are generally coated and labeled with linkers that can make them water soluble and chemically functional. They may be coated individually or a few of them are embedded in a shared matrix. In some embodiments, the MNP's may be replaced by magnetic nanorods, magnetic nanowires, etc.

In accordance with various embodiments, an MNP can be attached to a second (nucleic acid) NA strand 320 via covalent binding or other similarly strong binding approach. The linking strategies may include but not limited to: (1) Streptavidin (or just avidin) - Biotin; (2) -NHS functionalized MNP to bind with -NH₂ functionalized second (nucleic acid) NA strand; (3) -Sulfo SMCC functionalized MNP to bind with -HS functionalized second NA strand; (4) antibody functionalized MNP to bind with its corresponding antigen/antibody labeled second NA strand. The MNP's may be free to leave the chip surface upon reaction.

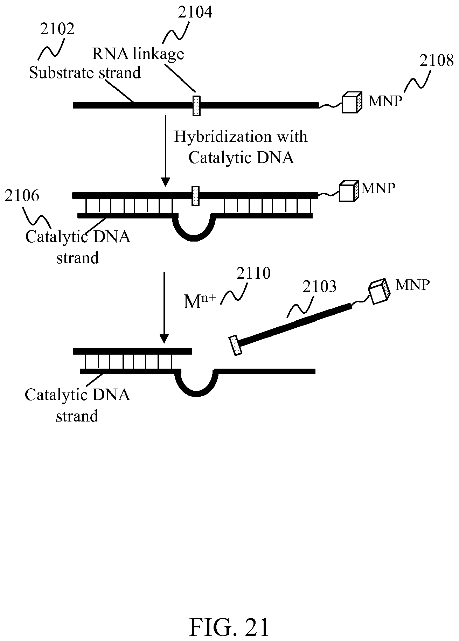

FIG. 21 illustrates an exemplary process for detecting metal ions based on substrate and catalytic DNA strands, according to an embodiment of the present teaching. In accordance with this embodiment, a ribonucleic acid (RNA) linked substrate strand 2102 is bound to a catalytic DNA strand 2106. The substrate strand 2102 has an MNP 2108 attached to its end. The substrate strand 2102 has an RNA linkage 2104, so that once a sample of liquid including some specific metal ion 2110 is in contact with the strands, the substrate strand 2102 can break at the RNA linkage 2104 to cause a fragment 2103 of the substrate strand 2102 and the MNP 2108 attached to the fragment to flow away. The reason is that a catalytic DNA strand 2106 works as an enzyme to promote a hydrolytic cleavage of the substrate strand 2102 and then release the fragment into the liquid sample. But the hydrolytic reaction rate is extremely slow and negligible unless and until the specific metal ions 2110 are present to significantly increases the activity of the catalytic DNA strand 2106 and make the hydrolytic reaction happen practically.

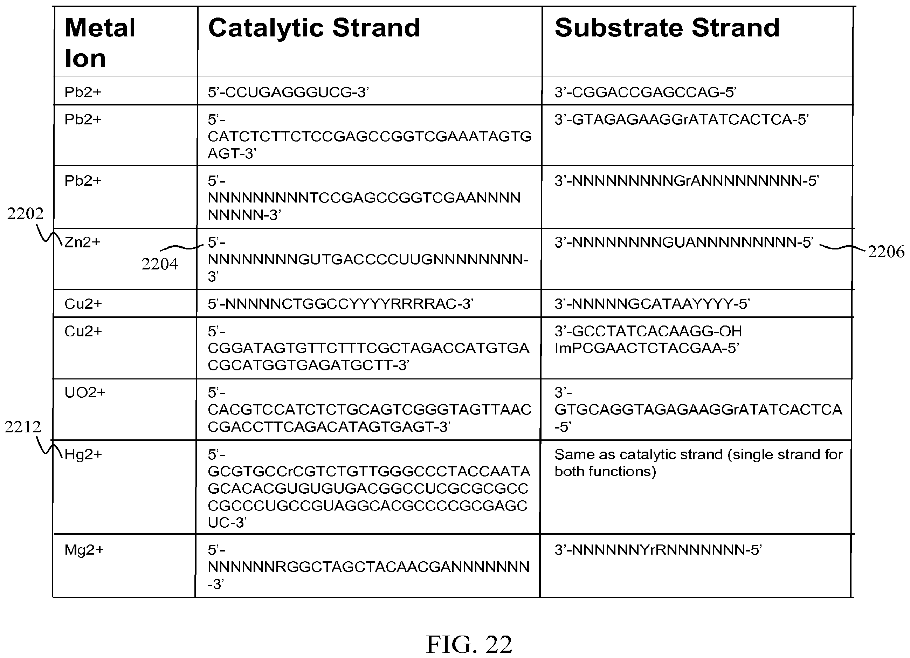

For carefully designed DNA or RNA combinations, metal ions can show high specificity to the reaction. Therefore, a type of metal ions can be detected selectively, because it is specific to a carefully designed DNA or RNA combination. FIG. 22 shows a table listing examples of metal ions to be detected, where each metal ion can be detected using a corresponding catalytic strand and a corresponding substrate strand listed in the same row as the metal ion, according to different embodiments of the present teaching. Each of the catalytic and substrate strands can be DNA or RNA or both. In one embodiment, the catalytic and substrate strands may be the same, e.g. the catalytic and substrate strands corresponding to the Hg2+ in the table. It can be understood by one skilled in the art that the methods and systems disclosed in the present teaching can be applied to detect any substance, including not limited to metal ions.

In accordance with various embodiments of the present teaching, both the catalytic strands and the substrate strands can be DNA, *i.e.,* a DNA/DNA coupling. This can be realized based on different methods. For DNAzyme where one catalytic strand is coupled with a substrate strand, while the catalytic strand is usually DNA, the substrate strand can be either DNA or RNA. In the situation of a structure-switching DNA, one strand is immobilized on chip surface (probe strand) and the other strand is initially hybridized with probe strand and will change its structure upon reacting with the target metal ion.

In the present teaching, the DNA's can keep their activity when being immobilized on a heterogeneous surface. This may be related to a distance from the DNA's to the chip surface and the length of DNA/RNA's, because the length affects their melting point, which should be high enough to make them stable at room temperature after hybridization.

The concentration boundary of detection for the technique in the present teaching is in a dynamic range, depending on the coating density of DNA as well as the sensitivity of sensor. Typically, the sensors can be designed to detect much diluted samples with a specified dynamic range. The lower concentration boundary of ion detection for this technique is about 1 ppb.

With pre-loaded DNA or RNA - MNP complex, the detection time of the technique in the present teaching depends mainly on two things: (1) chemical reaction time and (2) the speed of MNP leaving the sensor. In examples according to the present teaching, the chemical reaction time is usually several seconds to several minutes while the speed of MNP leaving is also seconds to minutes since MNP's only need to move away by about 200 nm. Therefore, the total detection time here with a subtractive-signal method can be expected to be within 5 minutes. In contrast, an additive-signal method needs incubate/wash for 1 to 2 hours.

The system in the present teaching is beneficially run in an aqueous environment. Non-aqueous sample like oil or organic may affect the detection principle and thus need to be pre-processed such that free metal ions can be released for detection.

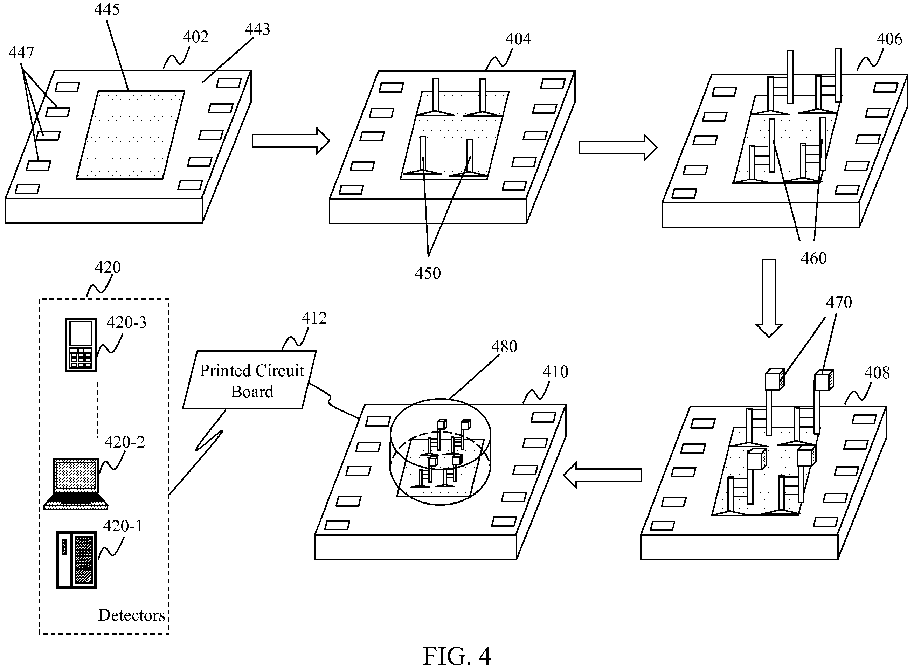

FIG. 4 illustrates an exemplary process for creating a sensing device for metal ion detection, according to an embodiment of the present teaching. In the present teaching, sensing device may also be referred to as a signal reading device. At 402, a chip having a base 443 and a plurality of contact pads 447 is obtained. A magnetic sensor 445, e.g. a GMR sensor, may be formed onto the base 443. In accordance with various embodiments, magnetic sensors other than GMR, e.g. anisotropic magnetoresistance (AMR) or tunneling giant magnetoresistance (TGMR), can be used as well. The magnetic sensor 445 is in the chip center and the plurality of contact pads 447 are around the chip edges. In accordance with one embodiment, the chip is protected against biological specimen by coating with an oxide layer or other nonmagnetic protection layer.

At 404, a first set of breakable strands 450 is attached to the magnetic sensor 445, e.g. via coating. At 406, each of the first set of breakable strands 450, wherein said breakable strands comprise functional nucleic acids having metal specificity, is bound to one of a second set of strands 460, e.g. via DNA/RNA coupling. At 408, each of the second set of strands 460 bound to the first set of breakable strands 460 is attached to a magnetic particle. For example, each of the second set of strands 460 is a biotin labeled DNA strand that can be bound to a streptavidin labeled MNP, via a biotin-streptavidin interaction.

At 410, a reaction well 480 is formed on the base 443, e.g. by attaching or gluing a plastic ring, which is typically Poly(methyl methacrylate) (PMMA), on the base 443. The reaction well 480 may have a round shape as shown in FIG. 4, or have other shapes like rectangular in another example. The reaction well 480 can prevent a liquid sample from escaping and keep concentration of ions constant by reducing evaporation rate of the liquid. The reaction well 480 may also keep the liquid sample around the magnetic sensor 445 and away from the contact pads 447. The contact pads 447 along the chip edges may be used for electrical connections to one or more detectors 420, via a printed circuit board (PCB) 412.

The one or more detectors 420 may be implemented on desktop computers 420-1, laptop computers 420-2, or a handheld mobile device 420-3, e.g. a tablet or a smart phone. The PCB 412 can be connected to a chip through either wires or pins for data collection. The PCB 412 is configured for signal collection, raw data processing, and communicating with a detector 420. The detector 420 can communicate with the PCB 412, via wired or wireless connection and software, e.g. a program on a computer or an application on a handheld device.

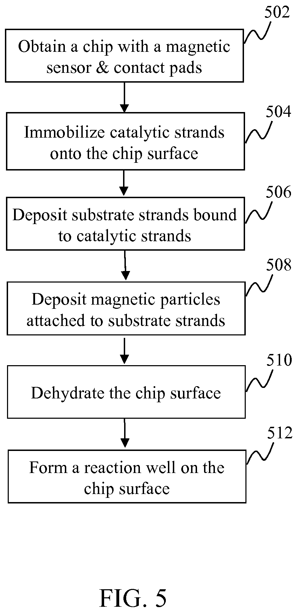

FIG. 5 is a flow chart of an exemplary process for creating a sensing device for metal ion detection, according to an embodiment of the present teaching. Starting at 502, a chip with a magnetic sensor and contact pads is obtained. At 504, catalytic strands are immobilized with respect to the chip surface. At 506, substrate strands are deposited onto the chip surface to be bound to the catalytic strands. At 508, magnetic particles are deposited onto the chip surface to be attached to the substrate strands. Optionally at 510, the chip surface is dehydrated to be ready for reaction well formation. Optionally at 512, a reaction well is formed on the chip surface.

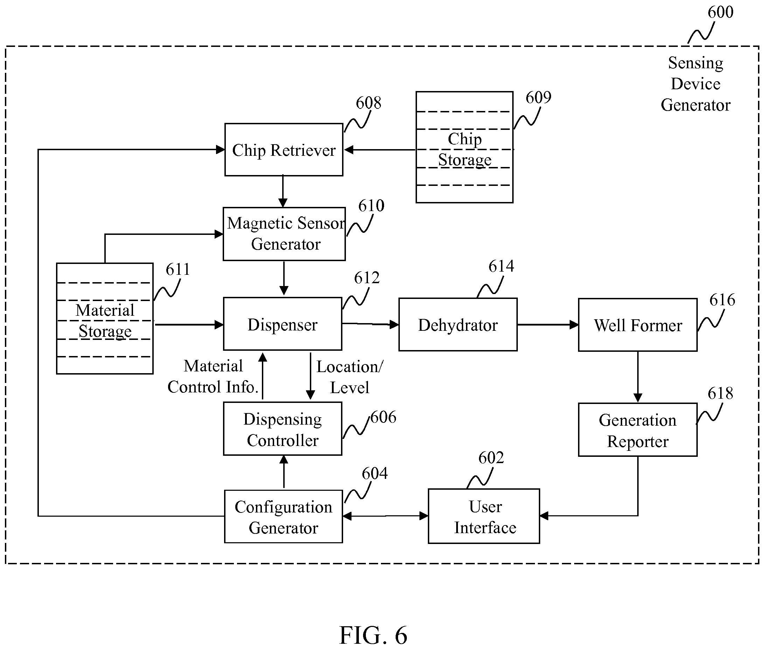

FIG. 6 illustrates an exemplary diagram of a sensing device generator 600, according to an embodiment of the present teaching. The sensing device generator 600 in this example includes a user interface 602, a configuration generator 604, a dispensing controller 606, a chip retriever 608, a magnetic sensor generator 610, a dispenser 612, a dehydrator 614, a well former 616, and a generation reporter 618.

The user interface 602 in this example is an interface for receiving an input from a user and displaying information to the user. For example, the user may input a request for generating a sensing device with some specific functions. The configuration generator 604 in this example generates configuration for a sensing device to be generated according to the user's request. In one example, the configuration generator 604 may generate and send questions and/or instructions to the user, via the user interface 602, for the user to input desired request information regarding generating the sensing device so that configuration may be properly generated. The configuration may include information related to what type of chip can be used for generating the sensing device, what type of material or material combinations may be deposited for generating the sensing device, and how and where should the material be deposited. The configuration may be sent to the chip retriever 608 and the dispensing controller 606.

The chip retriever 608 in this example retrieves a chip from chip storage 609 in the sensing device generator 600, according to the configuration received from the configuration generator 604. The chip storage 609 may store various types of chips. Different chips may comprise different materials; and have different shapes and/or different sizes. In one example, a chip stored in the chip storage 609 already has contact pads around its edge. In another example, the chip retriever 608 may retrieve a chip without contact pads and then form contact pads on it.

The magnetic sensor generator 610 in this example generates a magnetic sensor, e.g. a GMR sensor as shown in FIG. 1, and form the magnetic sensor onto a base of the chip retrieved by the chip retriever 608. In one example, the magnetic sensor generator 610 may retrieve material needed for the magnetic sensor generation, from material storage 611 in the sensing device generator 600. The material storage 611 may store various materials, including ferromagnetic and nonmagnetic materials for the magnetic sensor generation, magnetic particles like MNP, and/or biological materials for deposition onto the chip, e.g. catalytic strands, substrate strands, etc. In one embodiment, a plurality of magnetic sensors can be formed on the same chip, where each magnetic sensor may be utilized to detect a specific metal ion by sensing a magnetic field created by magnetic particles close to the magnetic sensor.

The dispenser 612 in this example obtains the magnetic sensor on the chip and dispenses materials onto the chip. In one example, the materials may be biological materials retrieved from the material storage 611. According to one embodiment of the present teaching, the dispenser 612 includes a depositing unit, e.g. one or more microarrayers, for depositing materials onto the chip at different locations and levels. The location is about a targeted depositing position on the chip. The level is about a step or phrase of the depositing process. For example, in FIG. 4, the first set of breakable strands 450 and the second set of strands 460 are deposited onto the chip at different levels, 404 and 406.

The dispenser 612 may send information about current location and level of the depositing unit in the dispenser 612 to the dispensing controller 606, and receives material control information from the dispensing controller 606. The material control information includes location and level information for the depositing unit in next time period and material to be retrieved and deposited in the next time period.

The dispensing controller 606 in this example receives current location and level of the depositing unit from the dispenser 612, receives configuration from the configuration generator 604, and generates the material control information based on the configuration and the current location and level of the depositing unit. In one example, if the configuration is for creating a sensing device to detect Zn2+ 2202 as shown in FIG. 22, and if the depositing unit just finishes depositing one catalytic strand 2204, the material control information may be generated to inform the depositing unit to deposit the same material again to form another catalytic strand 2204, at next location in next time period. In another example, if the configuration is for creating a sensing device to detect Zn2+ 2202 as shown in FIG. 22, and if the depositing unit finishes depositing all catalytic strands 2204 required for the sensing device, the material control information may be generated to inform the depositing unit to deposit a new material to form a substrate strand 2206, at next location in next time period. In one embodiment, the depositing unit can deposit all materials needed at one location before moving to another location. In another embodiment, there may be multiple depositing units in the dispenser 612 so that they can work cooperatively at the same time to deposit different materials or at different locations.

Once the dispenser 612 finishes dispensing and depositing materials onto the chip, the dehydrator 614 may perform dehydration on the chip surface so that a reaction well may be formed on the chip surface by the well former 616. As described before, the reaction well formation is optional and may prevent a liquid sample from escaping and keep concentration of ions constant by reducing evaporation rate of the liquid. The generation reporter 618 in this example generates a report about the sensing device generation and sends the report to the user interface 602 for the user to view.

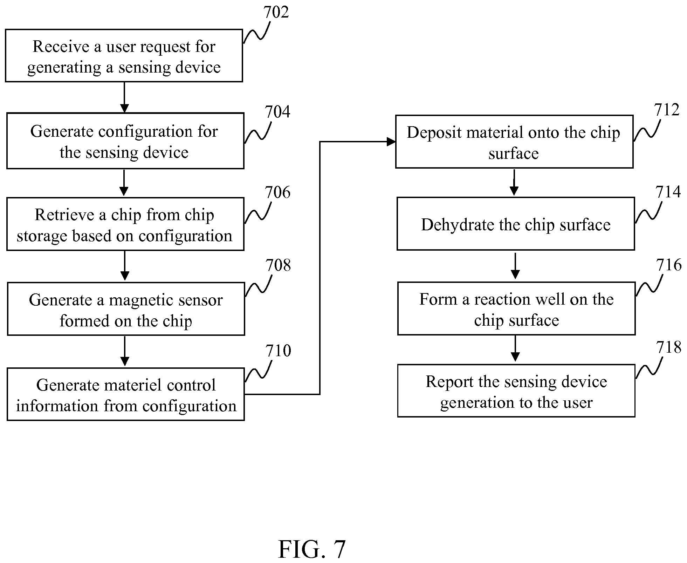

FIG. 7 is a flowchart of an exemplary process performed by a sensing device generator, e.g., the sensing device generator 600 in FIG. 6, according to an embodiment of the present teaching. Starting at 702, a user request is received for generating a sensing device. At 704, configuration for the sensing device is generated. At 706, a chip is retrieved from chip storage based on the configuration. At 708, a magnetic sensor is generated and formed on the chip.

At 710, material control information is generated based on configuration to determine the material to be deposited onto the chip. At 712, material is retrieved and deposited onto the chip surface according to the material control information. It can be understood by one skilled in the art that, 710 and 712 may be performed iteratively until all materials needed have been deposited onto the chip surface. The chip is then dehydrated optionally at 714 and has a reaction well formed on its surface optionally at 716. At 718, a report is generated and sent to the user regarding the sensing device creation, in response to the user request. It can be understood that, in accordance with various embodiments, at least some of the above mentioned steps in FIG. 7 may happen without following the sequence shown in FIG. 7.

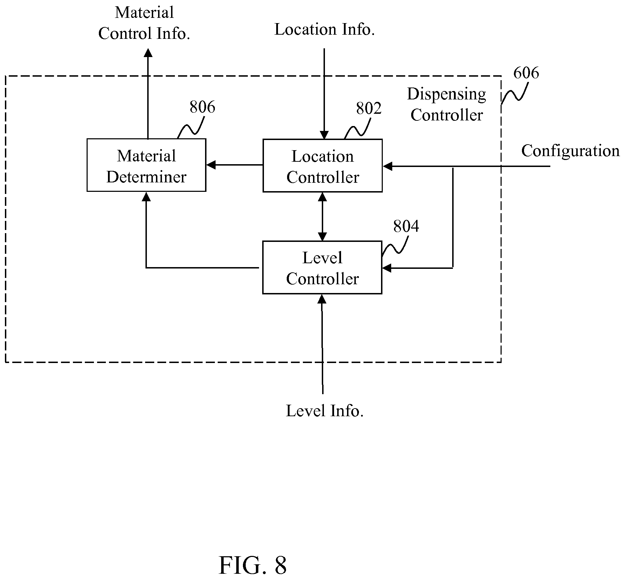

FIG. 8 illustrates an exemplary diagram of a dispensing controller 606 in a sensing device generator, according to an embodiment of the present teaching. The dispensing controller 606 may be in the sensing device generator 600 shown in FIG. 6. The dispensing controller 606 in this example includes a location controller 802, a level controller 804, and a material determiner 806.

The location controller 802 in this example controls next location for the deposition by the dispenser 612 in next time period. The next location can be determined according to configuration received from the configuration generator 604 and location information from the dispenser 612 that indicates a current location of the deposition. The level controller 804 in this example controls level for the deposition by the dispenser 612 in next time period. The level in next time period can be determined according to configuration received from the configuration generator 604 and level information from the dispenser 612 that indicates a current level of the deposition. In one embodiment, the location controller 802 and the level controller 804 may exchange information to perform in a more efficient manner. For example, the location controller 802 can pass the location information to the level controller 804 so that the level controller 804 can make a better and faster decision about whether to move to a new level for deposition in next time period.

The material determiner 806 in this example determines material(s) to be used for deposition at a controlled location and level in next time period. In one embodiment, the location controller 802 and/or the level controller 804 can pass the configuration to the material determiner 806, so that the material determiner 806 can determine the material based on the configuration with respect to the location and level in next time period determined by the location controller 802 and the level controller 804 respectively. The material determiner 806 may generate material control information about the material to use and the location/level in next time period. The material determiner 806 can send the material control information to the dispenser 612 for material deposition.

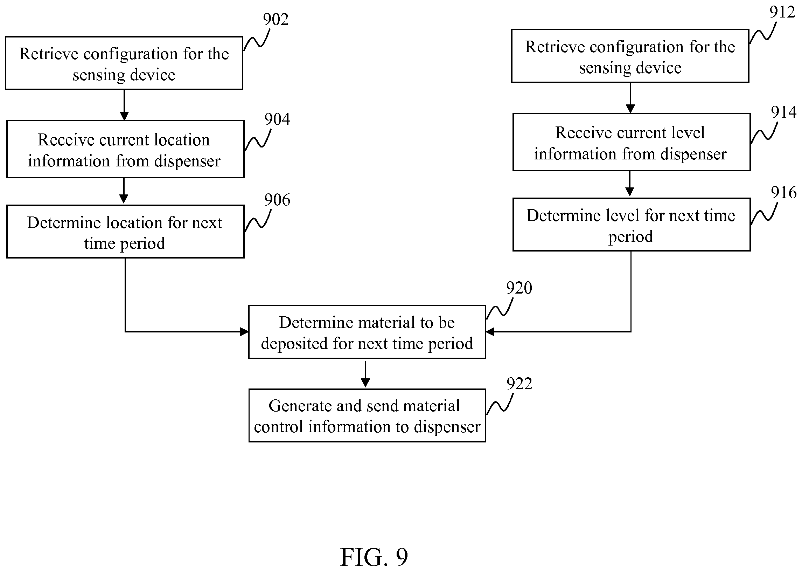

FIG. 9 is a flowchart of an exemplary process performed by a dispensing controller, e.g., the dispensing controller 606 in FIG. 8, according to an embodiment of the present teaching. At 902, configuration for the sensing device is retrieved. At 904, current location information is received from the dispenser. At 906, location for deposition in next time period is determined. At 912, configuration for the sensing device is retrieved. At 914, current level information is received from the dispenser. At 916, level for deposition in next time period is determined. In one example, the process 912-916 is performed in parallel to the process 902-906, as shown in FIG. 9. In another example, the process 912-916 is performed in serial to (either before or after) the process 902-906.

At 920, material(s) to be deposited for the next time period is determined. At 922, material control information is generated and sent to the dispenser for deposition in the next time period. It can be understood that, in accordance with various embodiments, at least some of the above mentioned steps in FIG. 9 may happen without following the sequence shown in FIG. 9.

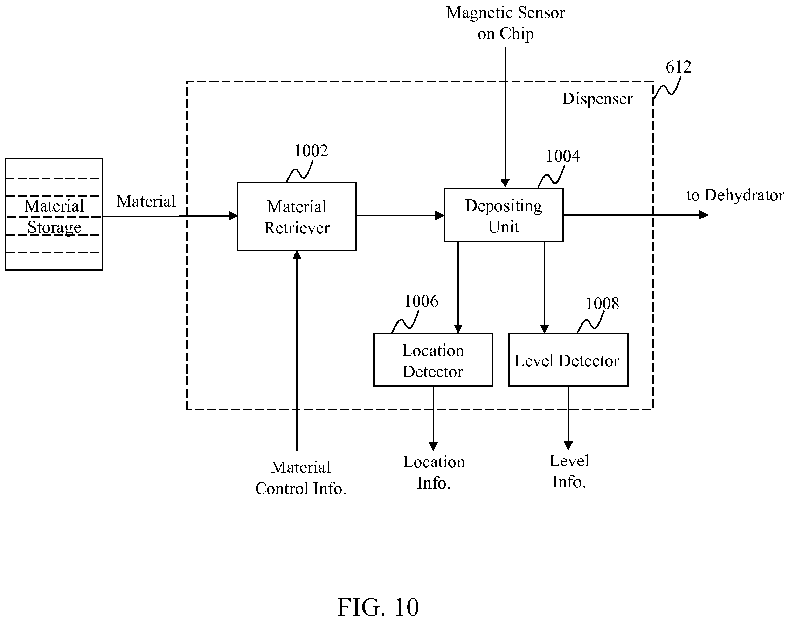

FIG. 10 illustrates an exemplary diagram of a dispenser 612 in a sensing device generator, according to an embodiment of the present teaching. The dispenser 612 may be in the sensing device generator 600 shown in FIG. 6. The dispenser 612 in this example includes a material retriever 1002, a depositing unit 1004, a location detector 1006, and a level detector 1008.

The material retriever 1002 in this example receives material control information from the dispensing controller 606 and retrieves material from the material storage according to the material control information. The material retriever 1002 may pass the retrieved material as well as the material control information to the depositing unit 1004.

The depositing unit 1004 in this example may obtain the magnetic sensor on the chip for dispensing, before the deposition starts. In one example, the depositing unit 1004 deposits material in a plurality of time periods. In each time period, the depositing unit 1004 may also obtain the material retrieved by the material retriever 1002 and receive material control information from the material retriever 1002. The depositing unit 1004 can determine where and how to deposit based on the material control information. At the end of each time period, the depositing unit 1004 sends information to the location detector 1006 and the level detector 1008 for determining the ending location and level. After the depositing unit 1004 finishes all required deposition in the plurality of time periods, the depositing unit 1004 can send the chip being deposited with materials to the dehydrator 614.

The location detector 1006 in this example detects location information of the depositing unit 1004 at the end of each time period and sends the location information to the dispensing controller 606. The level detector 1008 in this example detects level information of the depositing unit 1004 at the end of each time period and sends the level information to the dispensing controller 606. As described before, the location and level information at the end of each time period will be utilized by the dispensing controller 606 to determine controlled location and level for deposition in next time period.

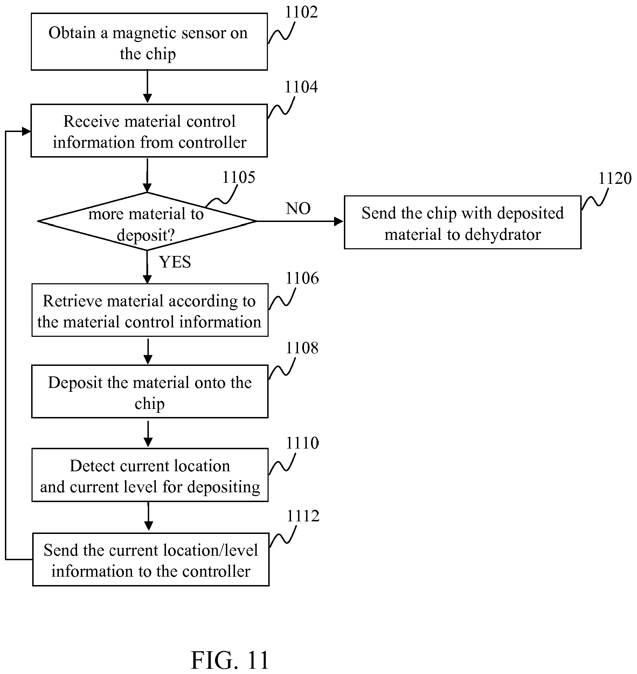

FIG. 11 is a flowchart of an exemplary process performed by a dispenser, e.g., the dispenser 612 in FIG. 10, according to an embodiment of the present teaching. Starting at 1102, a magnetic sensor on the chip is obtained. At 1104, material control information is received from the dispensing controller 606. At 1105, whether there is any more material to deposit is determined, e.g., based on the material control information. If not, at 1120, the chip is sent to the dehydrator 614 with deposited material.

Otherwise, if there is more material to deposit, the process goes to 1106, where material is retrieved according to the material control information. Then, at 1108, the material is deposited onto the chip. The current location and level for depositing are detected at 1110 and sent to the dispensing controller 606 at 1112. Then, the process moves back to 1104 to receive material control information for next time period. It can be understood that, in accordance with various embodiments, at least some of the above mentioned steps in FIG. 11 may happen without following the sequence shown in FIG. 11.

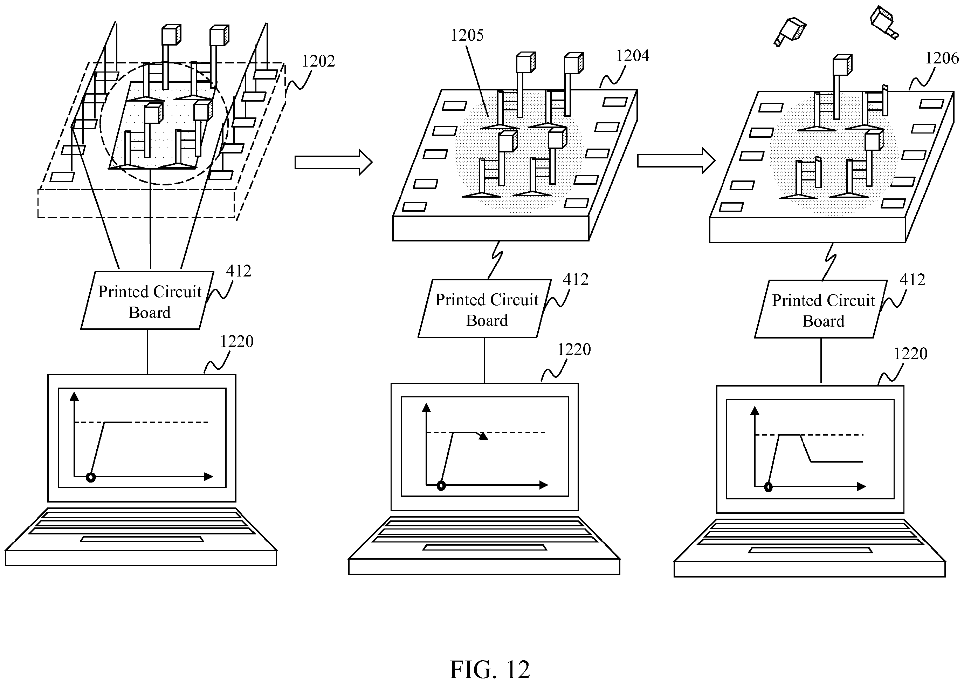

FIG. 12 illustrates an exemplary process for detecting a metal ion in a sample based on a subtractive-signal method, according to an embodiment of the present teaching. At 1202, a sensing device has been created with a magnetic sensor, a first set of breakable strands, wherein said breakable strands comprise functional nucleic acids having metal specificity, a second set of strands, magnetic particles, and contact pads, similar to the phrase 410 shown in FIG. 4 and described before. In this example, the reaction well is referred by a circle on the chip surface for simplicity. In this example, the contact pads and the magnetic sensor are connected to the PCB 412, via some interface, e.g. a wire connection, a wireless connection, pins like USB, etc. The PCB 412 is configured for signal collection from the chip, raw data processing, and communicating with a detector implemented on a machine, e.g. a laptop 1220 in this example. As described before, because each strand pair has a corresponding magnetic particle immobilized in proximity to the magnetic sensor, at 1202, a level of magnetic field sensed by the magnetic sensor is at a maximum level or a fixed large level. Hence, at 1202, a signal level displayed on the laptop 1220, which may be a voltage or current level derived from the level of the magnetic field, is also at a maximum level or a fixed large level.

At 1204, a liquid sample 1205 including a metal ion to be detected was just added onto the chip. The metal ion is specific to the strands on the sensing device and starts to cause some strands to cleave and release some magnetic particles into the liquid sample. Once released, the magnetic particles will flow away from the sensor and hence cannot contribute to the magnetic field in proximity to the sensor any more. Therefore, at 1204, the signal level displayed on the laptop 1220 starts to drop.

In one embodiment, some liquid other than the sample needs to be added onto the sensing device to activate the sensing device before adding the sample. In another embodiment, the liquid sample 1205 itself can activate the sensing device directly. An exemplary activation process before adding the sample is like the following: a small volume of buffer liquid is added into the reaction well to make the catalytic strands, the substrate strands and the MNP's wet and activated for magnetic signal producing; control electronics of the sensing device then sends biasing current to the GMR sensors, which are experiencing a magnetic field generated by an electromagnet powered and controlled by the electronics as well; and the magnetic signal (e.g. voltage signal) is then at its maximum level before the sample is added.

At 1206, the cleavage reactions have finished in the sample. At this stage, a subset of the magnetic particles has become released and flowed away from the magnetic sensor. Therefore, at 1206, the signal level displayed on the laptop 1220 has dropped to a lower level and stabilized there. The reduction of the signal level displayed at 1220 can be converted to a level of metal ion in the sample, through e.g. a standard metal ion level to signal reduction curve generated based on some training data.

While the cleavage reactions are not reversible directly, the sensing device may be rechargeable and reusable based on the following exemplary steps: (1) heat the chip up to relatively high temperature (*e.g*., 100 °C) to denature all DNA/RNA strands and wash off all second NA strands or their leftovers; (2) re-introduce fresh second NA strands to hybridize with the first NA strands to rebuild the structure; (3) label the second NA strands with MNP's.

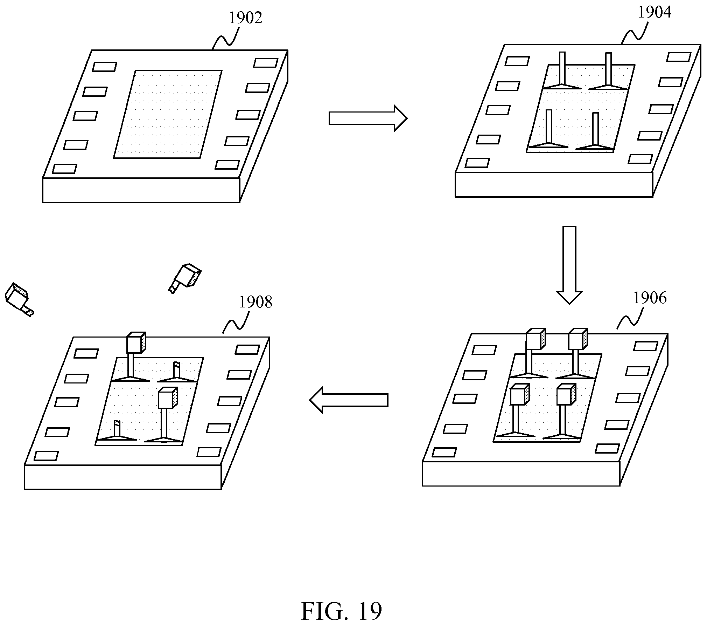

In accordance with one embodiment of the present teaching, a similar metal ion detection process can be achieved with a sensing device having only one set of breakable strands. FIG. 19 illustrates an exemplary process for detecting a metal ion with magnetic particles and one set of breakable strands, wherein said breakable strands comprise functional nucleic acids having metal specificity, according to an embodiment of the present teaching. At 1902, a magnetic sensor has been formed onto a base of a chip having a plurality of contact pads, in a similar manner as in the stage 402 in FIG. 4. At 1904, a set of breakable strands is attached to the magnetic sensor, e.g. via coating, in a similar manner as in the stage 404 in FIG. 4. At 1906, each of the set of breakable strands is attached to a magnetic particle, without a second set of strands. At 1908, a sample of material including a metal ion to be detected is added onto the chip, so that at least some of the set of breakable strands may break due to the presence of the metal ion in the sample. Thus, the magnetic particle attached to each broken strand is no longer in proximity to the magnetic sensor, which causes a reduction of the signal level of the magnetic field sensed by the magnetic sensor. Similar to FIG. 12, the reduction of the signal level here can be converted to a level of metal ion in the sample, through e.g. a standard metal ion level to signal reduction curve generated based on some training data.

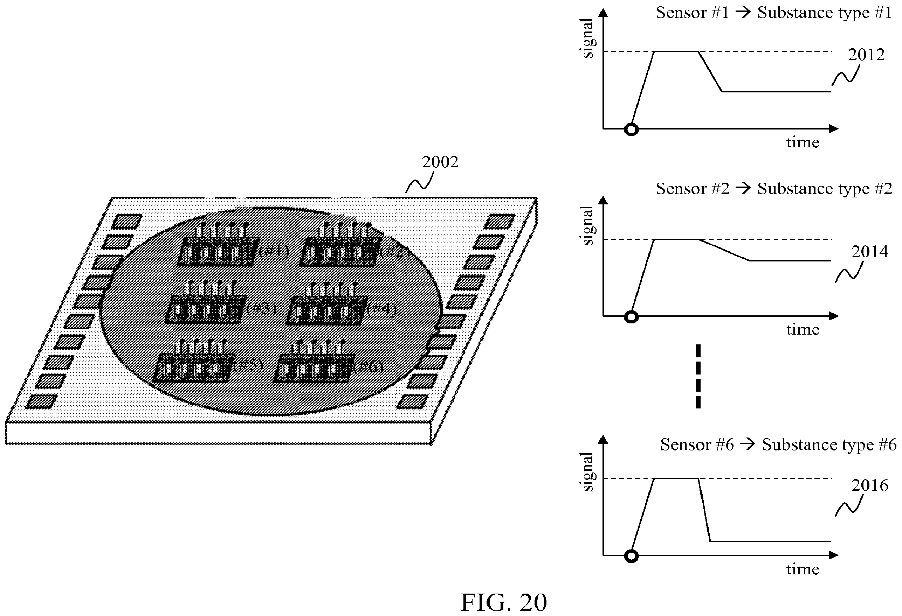

In accordance with one embodiment of the present teaching, a plurality of magnetic sensors can be formed on the same chip, where each magnetic sensor may be utilized to detect a specific metal ion by sensing a magnetic field created by magnetic particles close to the magnetic sensor. FIG. 20 illustrates exemplary signal changing processes for a multi-metal ion detection using multiple magnetic sensors on a same device 2002, according to an embodiment of the present teaching.

As shown in FIG. 20, the sensing device 2002 in this example includes six magnetic sensors formed on the base of the sensing device 2002, within a same reaction well. Each of the six magnetic sensors can sense a magnetic field in proximity to the sensor and the sensed level of the magnetic field can be used to detect a different metal ion simultaneously. Because magnetic field strongly and inversely depends on distance, each of the well-separated magnetic sensors as shown in FIG. 20 will not be affected by the MNP's attached to other magnetic sensors next to it.

The six different metal ions are all contained in a same sample to be added onto the sensing device 2002. Each magnetic sensor has some breakable strands, wherein said breakable strands comprise functional nucleic acids having metal specificity, attached thereon corresponding to the different metal ion to be detected via the magnetic sensor. For example, magnetic sensor #1 has a set of breakable strands #1 that can react due to presence of metal ion type #1 and cause a magnetic field in proximity to the magnetic sensor #1 to reduce to a lower level. Thus, a signal reduction sensed by the magnetic sensor #1 representing the magnetic field can be used to estimate or detect a level of the metal ion type #1 in the sample. Similarly, each magnetic sensor #n can sense a reduction of the magnetic field in proximity to the magnetic sensor #n and the reduction can be used to estimate or detect a level of the metal ion type #n in the sample, simultaneously.

The signal-time curve 2012 in this example illustrates a signal changing process for magnetic sensor #1. The signal level displayed by the signal-time curve 2012 may be a voltage or current level derived from the level of the magnetic field. As shown in FIG. 20, the signal-time curve 2012 initially increases to a fixed maximum level when the sensing device 2002 is ready for metal ion detection. After the sample including six metal ions is added, the signal level of the signal-time curve 2012 gradually dropped to a lower level and stabilized there. Similarly, the curve 2014 illustrates a signal changing process for magnetic sensor #2; and the curve 2016 illustrates a signal changing process for magnetic sensor #6. As shown in FIG. 20, different magnetic sensors can have different signal dropping rate after the sample is added.

The detection of each metal ion shown in FIG. 20 is based on a subtractive-signal method. In accordance with one embodiment of the present teaching, at least one of the six magnetic sensors in FIG. 20 can be designed based on an additive-signal method. For example, magnetic sensor #3 may have a set of breakable strands #3 that can react due to presence of metal ion type #3 and cause a magnetic field in proximity to the magnetic sensor #1 to increase to a higher level. Thus, a signal increase sensed by the magnetic sensor #3 representing the magnetic field can be used to estimate or detect a level of the metal ion type #3 in the sample.

The sensing device 2002 can detect multiple analytes simultaneously because GMR is a proximity sensor. One GMR sensor group cannot detect the MNP's on the neighboring sensor groups if they are sufficiently spatially distanced. This GMR-MNP method will not cause cross-talk concerns when the spacing between two sensor groups is usually on the order of microns and the GMR can generally only detect MNP's within about 200 nm.

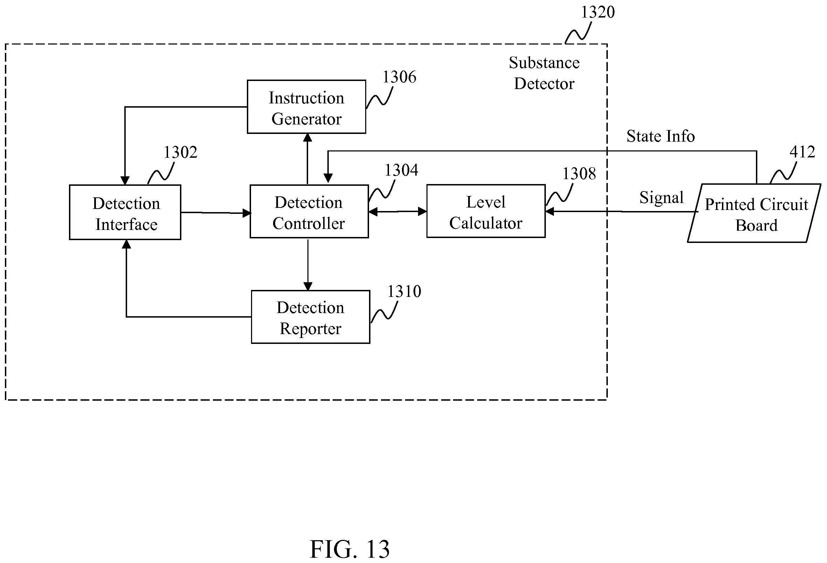

FIG. 13 illustrates an exemplary diagram of a metal ion detector 1320, according to an embodiment of the present teaching. The metal ion detector 1320 may be part of one of the detectors 420, as shown in FIG. 4. The metal ion detector 1320 in this example includes a detection interface 1302, a detection controller 1304, an instruction generator 1306, a level calculator 1308, and a detection reporter 1310.

The detection interface 1302 in this example is an interface for receiving an input from a user and displaying information to the user regarding metal ion detection. For example, the user may input a request for detecting a level of a metal ion in a material. The detection controller 1304 in this example controls the detection by coordinating different units in the metal ion detector 1320. For example, after a user inputs a request for detecting a level of a metal ion in a material, the detection controller 1304 may initiate the instruction generator 1306 to generate a user instruction for the user to perform some actions like plug in a magnetic sensor, add the material onto the sensor, etc. The detection controller 1304 may also verify a state of the magnetic sensor via a PCB 412, so that the detection controller 1304 can check whether the magnetic sensor has been properly connected, whether the material has been added, and/or whether it is ready to detect the metal ion in the material, etc. The detection controller 1304 may send control information to the level calculator 1308 to calculate or estimate a level of the metal ion, receive the estimated level from the level calculator 1308, and initiate the detection reporter 1310 to generate a detection report to be provided to the user.

The instruction generator 1306 in this example is configured for generating and providing instructions to the user via the detection interface 1302. The instructions may comprise: input or select one or more metal ions to be detected in a sample, input or select an amount of the sample to be added, connect to the metal ion detector a corresponding sensing device for sensing the one or more metal ions, check again whether the corresponding sensing device has been connected, add the sample onto the sensing device, read and download the detection report, and/or whether an additional detection is needed.

The level calculator 1308 in this example receives, via the PCB 412, a signal, e.g. a signal representing a level of a magnetic field sensed by a magnetic sensor on the connected corresponding sensing device. When a plurality of metal ions is requested to be detected in a same sample, a plurality of signals, each of which representing a level a magnetic field sensed by one of a plurality of magnetic sensors on the same connected corresponding sensing device. The level calculator 1308 may calculate and estimate a level of the metal ion in the sample based on levels of the signal received from the corresponding sensing device before and after the sample is added, and/or calculation control information received from the detection controller 1304.

The detection reporter 1310 in this example generates a detection report including a detection result about the metal ion detection requested by the user. The detection result may then be displayed to the user via the detection interface 1302, as a response to the detection request from the user.

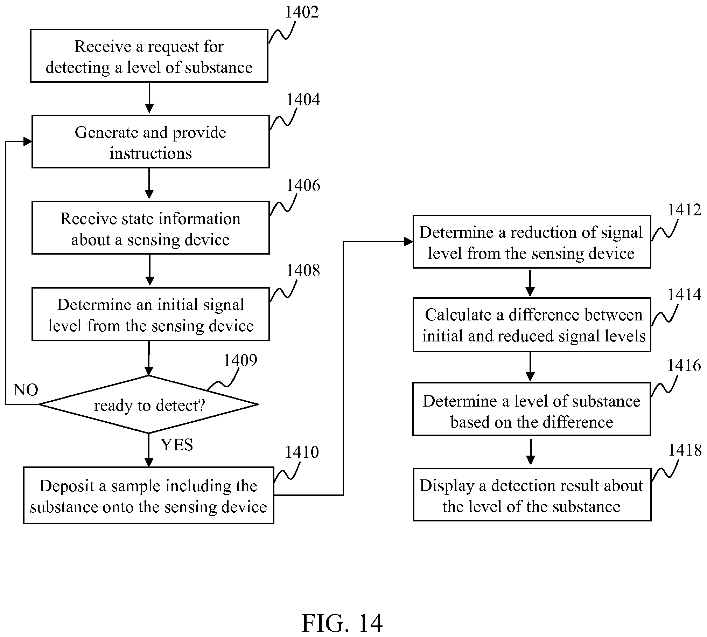

FIG. 14 is a flowchart of an exemplary process performed by a metal ion detector, e.g., the metal ion detector 1320 in FIG. 13, according to an embodiment of the present teaching. Starting at 1402, a request for detecting a level of metal ion is received from a user. At 1404, one or more instructions are generated and provided to the user. At 1406, state information about a corresponding sensing device for detecting the metal ion is received. At 1408, an initial signal level is determined from the sensing device, e.g. before the sample including the metal ion is added onto the sensing device.

At 1409, whether it is ready to detect the metal ion is checked, e.g. based on the state information about the sensing device. If not, the process goes back to 1404 to generate and provide more instructions to the user to collect more information or instruct the user to perform more actions. Otherwise, if it is ready to detect the metal ion, the process moves on to 1410, where a sample including the metal ion is deposited onto the sensing device. It can be understood by one skilled in the art that that 1410 may be either performed by the user under an instruction generated and provided by the metal ion detector 1320, or performed by the sensing device connected to the metal ion detector 1320. For example, after the user inputs, via the detection interface 1302, to select 0.01 mL sample to be added to the sensing device, the user may add enough sample into a funnel associated with the sensing device. The sensing device may control the funnel to deposit exactly 0.01 mL sample onto the sensing device.

At 1412, a reduction of signal level is determined from the sensing device, e.g. due to the presence of the metal ion in the sample. At 1414, a difference between the initial and reduced signal levels is calculated. The difference may be proportional to an amount of the metal ion in the sample. At 1416, a level of the metal ion is determined based on the difference, given the amount of the sample added onto the sensing device. At 1418, a detection result about the level of the metal ion is generated and displayed to the user, as a response to the request from the user. It can be understood that, in accordance with various embodiments, at least some of the above mentioned steps in FIG. 14 may happen without following the sequence shown in FIG. 14.

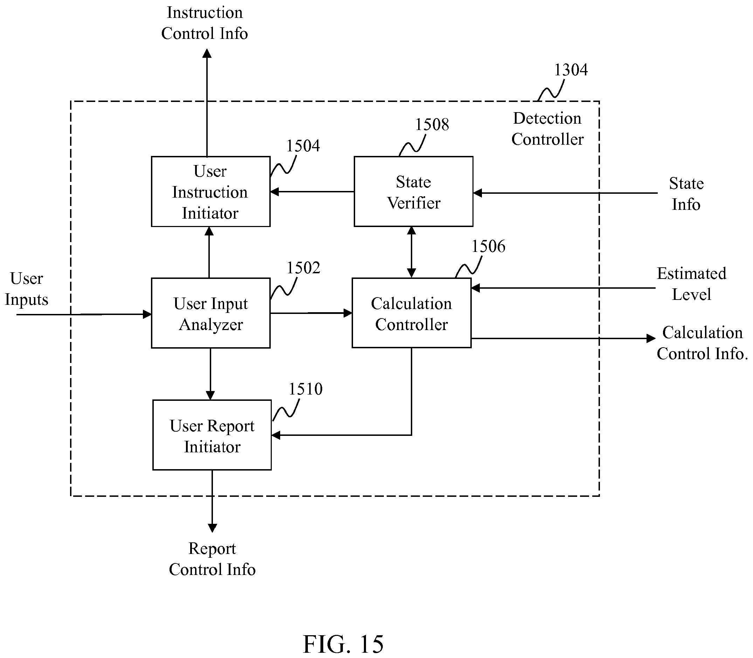

FIG. 15 illustrates an exemplary diagram of a detection controller 1304 in a metal ion detector, e.g., the metal ion detector 1320 in FIG. 13, according to an embodiment of the present teaching. The detection controller 1304 in this example includes a user input analyzer 1502, a user instruction initiator 1504, a calculation controller 1506, a state verifier 1508, and a user report initiator 1510. The user input analyzer 1502 in this example analyzes user inputs received from a user via the detection interface 1302 and sends information about the analyzed inputs to other units in the detection controller 1304. In one example, if the user inputs a request to detect a metal ion Hg2+ in a liquid sample, the user input analyzer 1502 may send information to the user instruction initiator 1504 for initiating further instructions like: input the amount of the liquid sample. In another example, if the user has input all required information for detecting the metal ion Hg2+ in a liquid sample, and perform all required actions following instructions provided by the metal ion detector 1320, the user input analyzer 1502 may send information to the calculation controller 1506 to control an estimate of the level of the metal ion at the level calculator 1308.

The user instruction initiator 1504 in this example is configured to initiate the instruction generator 1306 to generate one or more instructions to the user. The one or more instructions are generated based on the information sent by the user input analyzer 1502 regarding user inputs or based on state verification from the state verifier 1508. The state verifier 1508 in this example may receive state information from a PCB 412 and verify a state of the sensing device connected for sensing the metal ion in the sample. For example, if the sensing device has not been connected or activated, the state verifier 1508 may inform the user instruction initiator 1504 to initiate the instruction generator 1306 to generate an instruction to remind the user to connect or activate the sensing device.

The calculation controller 1506 in this example is configured to generate and send calculation control information to the level calculator 1308 to calculate an estimated level of the metal ion in the sample. The calculation control information may be generated based on information sent by the user input analyzer 1502 and state verification sent by the state verifier 1508. The calculation controller 1506 may also receive the estimated level from the level calculator 1308 and send the estimated level to the user report initiator 1510 for generating a detection report. In one embodiment, after the estimated level is received at the calculation controller 1506, the calculation controller 1506 may inform the state verifier 1508 to double check the state of the sensing device, e.g. because the estimated level is lower or higher than a predetermined threshold. In another embodiment, after the estimated level is received at the calculation controller 1506, the calculation controller 1506 may generate information about whether the estimated level is lower or higher than a predetermined threshold, e.g. a threshold associated with some law or regulation. The information may be sent to the user report initiator 1510 for generating a detection report. The user report initiator 1510 in this example can initiate the detection reporter 1310 for generating a detection report to include information about: the level of the metal ion in the sample, whether the level is high or low compared to a threshold related to some law or regulation, and/or suggestions regarding usage of the material, etc.

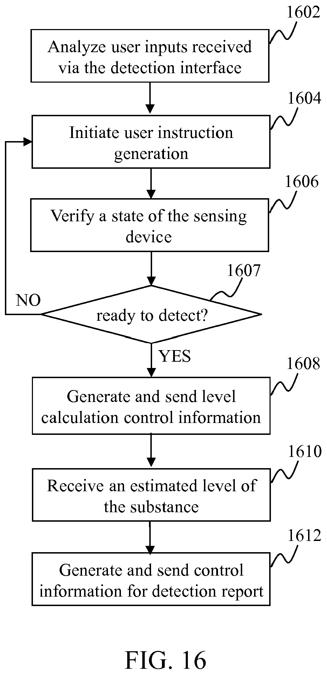

FIG. 16 is a flowchart of an exemplary process performed by a detection controller, e.g., the detection controller 1304 in FIG. 15, according to an embodiment of the present teaching. Starting at 1602, one or more user inputs received via the detection interface are analyzed, regarding a detection of a metal ion in a sample. At 1604, user instruction generation is initiated based on the analyzed inputs. At 1606, a state of the corresponding sensing device for sensing the metal ion to be detected is verified.

At 1607, whether it is ready to detect the metal ion is checked, e.g. based on the state of the sensing device. If not, the process goes back to 1604 to initiate generating more user instruction(s) to the user to collect more information or instruct the user to perform more actions. Otherwise, if it is ready to detect the metal ion, the process moves on to 1608, where control information related to level calculation of the metal ion is generated and sent, e.g. to the level calculator 1308. At 1610, an estimated level of the metal ion is received, e.g. from the level calculator 1308. At 1612, control information is generated and sent, e.g. to the detection reporter 1310, for generating a detection report. It can be understood that, in accordance with various embodiments, at least some of the above mentioned steps in FIG. 16 may happen without following the sequence shown in FIG. 16.

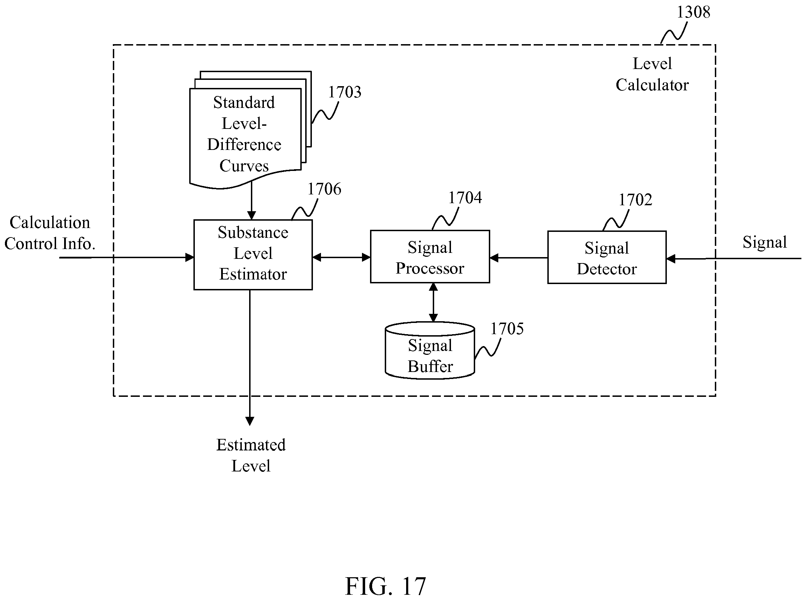

FIG. 17 illustrates an exemplary diagram of a level calculator 1308 in a metal ion detector, e.g., the metal ion detector 1320 in FIG. 13, according to an embodiment of the present teaching. The level calculator 1308 in this example includes a signal detector 1702, a signal processor 1704, and a metal ion level estimator 1706. The signal detector 1702 in this example detects a signal transmitted from a corresponding sensing device, via a PCB 412. For example, a sensing device including a magnetic sensor and strands listed in the row of Hg2+ 2212 in FIG. 22 can be used as a corresponding sensing device when the user requests to detect Hg2+ in a sample. The signal may be an electrical signal, e.g. a voltage or current signal, representing a level of a magnetic field in proximity to and sensed by the magnetic sensor.

The signal processor 1704 may process the signal detected at 1702, by e.g. removing noise, whitening noise, reducing interference, reducing matrix effect, and/or transferring the signal from one type to another. The signal processor 1704 may also save the processed signal into a signal buffer 1705 in the level calculator 1308, so that the saved signal may be retrieved later for calculation. For example, before the sample is added onto the sensing device, the signal processor 1704 may process a signal detected by the signal detector 1702 that represents a level of the magnetic field in proximity to and sensed by the magnetic sensor, and save an initial signal level of the signal into the signal buffer 1705. After the sample is added onto the sensing device, the signal processor 1704 may process the same signal that represents a level of the magnetic field in proximity to and sensed by the magnetic sensor, and save a reduced signal level of the signal into the signal buffer 1705. The signal processor 1704 may then retrieve both the initial and reduced levels and calculate a difference between them. In one embodiment, the signal processor 1704 may only save the initial level and determine the difference based on the reduced level and the retrieved initial level. In another embodiment, the information about whether the sample is added onto the sensing device can be included in the calculation control information that is received from the detection controller 1304 and can be passed from the metal ion level estimator 1706 to the signal processor 1704.

The metal ion level estimator 1706 in this example receives the calculation control information, e.g. from the detection controller 1304, receives the difference between the two levels from the signal processor 1704, and estimates a level of the metal ion accordingly. The metal ion level estimator 1706 may select and retrieve one of a plurality of standard level-difference curves 1703 stored in the level calculator 1308. Each of the standard level-difference curves 1703 is a standard curve representing a predetermined relationship between a level of a metal ion in a sample and a difference from two levels, where the two levels are an initial signal level before the sample is added and a reduced signal level after the sample is added. For example, the metal ion level estimator 1706 selects a corresponding standard curve 1703 based on the calculation control information including e.g. the type of the metal ion, the amount of the sample added, the type of the sample, the temperature of the sample, the type of the signal, and/or the detection time. Each standard curve was generated based on known training data. For example, a plurality of liquid samples each of which including a different known level of Hg2+ are added onto the sensing device respectively to determine a plurality of signal level differences each of which corresponds to one known level of Hg2+. Based on some signal smoothing technique, a standard level-difference curve can be generated to represent a relationship between the level of Hg2+ in the liquid sample and a signal level difference calculated at the signal processor 1704.

Accordingly, the metal ion level estimator 1706 can determine or estimate a level of the metal ion in the sample, based on the calculated difference at the signal processor 1704 and a known relationship between the difference and the level of the metal ion from the selected standard level-difference curve 1703. The metal ion level estimator 1706 may send the estimated level to the detection controller 1304 to collect information to generate a detection report. In one embodiment, the metal ion level estimator 1706 determines an amount of the metal ion instead of a level of the metal ion, based on some standard amount-difference curve, and the calculation controller 1506 in the detection controller 1304 can determine the level of the metal ion based on the estimated amount at the metal ion level estimator 1706 and the amount of the sample input by the user.

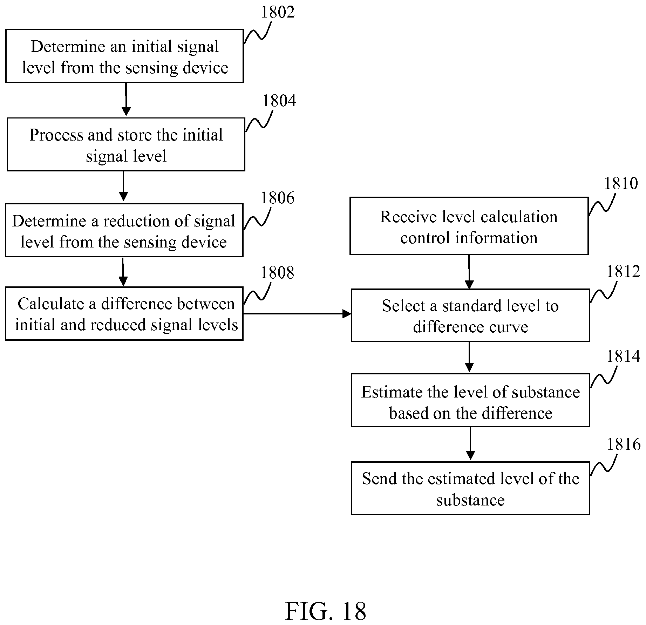

FIG. 18 is a flowchart of an exemplary process performed by a level calculator, e.g., the level calculator 1308 in FIG. 17, according to an embodiment of the present teaching. Starting at 1802, an initial signal level is determined from the sensing device. At 1804, the initial signal level is processed and stored, e.g. in a signal buffer. At 1806, a reduction of signal level is determined from the sensing device, e.g. due to deposition of the sample including the metal ion to be detected. At 1808, a difference between initial and reduced signal levels is calculated, and the process moves on to 1812. At 1810, level calculation control information is received, e.g. from the detection controller 1304, and the process moves to 1812. As describe above, the calculation control information may comprise information about the type of the metal ion, the amount of the sample added, the type of the sample, the temperature of the sample, the type of the signal, and/or the detection time. It can be understood by one skilled in the art that process 1802-1808 and process 1810 may be performed either in parallel as shown in FIG. 18 or in serial in accordance with other embodiments of the present teaching.

At 1812, a standard level-difference curve is selected, e.g. based on the calculation control information. At 1814, the level of metal ion is estimated based on the difference and the selected standard level-difference curve. At 1816, the estimated level of the metal ion is sent, e.g. to the detection controller 1304. It can be understood that, in accordance with various embodiments, at least some of the above mentioned steps in FIG. 18 may happen without following the sequence shown in FIG. 18.

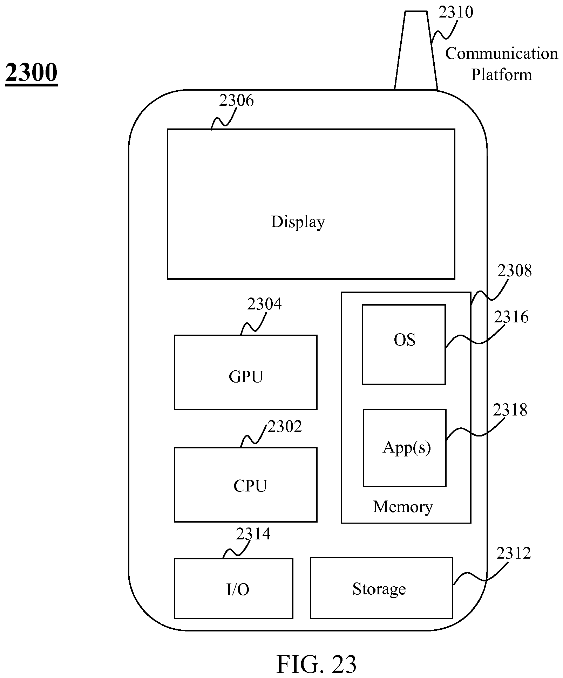

FIG. 23 depicts a general mobile device architecture on which the present teaching can be implemented. In this example, the detector 420 is implemented on a mobile device 2300, including but is not limited to, a smart phone, a tablet, a music player, a handled gaming console, a GPS receiver. The mobile device 2300 in this example includes one or more central processing units (CPUs) 2302, one or more graphic processing units (GPUs) 2304, a display 2306, a memory 2308, a communication platform 2310, such as a wireless communication module, storage 2312, and one or more input/output (I/O) devices 2314. Any other suitable component, such as but not limited to a system bus or a controller (not shown), may also be included in the mobile device 2300. As shown in FIG. 23, a mobile operating system 2316, e.g., iOS, Android, Windows Phone, etc., and one or more applications 2318 may be loaded into the memory 2308 from the storage 2312 in order to be executed by the CPU 2302. The applications 2318 may include a web browser or any other suitable mobile search apps. Execution of the applications 2318 may cause the mobile device 2300 to perform some processing as described before. For example, the display of a detection result is made by the GPU 2304 in conjunction with the display 2306. User inputs for detecting a metal ion are received via the I/O devices 2314.

To implement the present teaching, computer hardware platforms may be used as the hardware platform(s) for one or more of the elements described herein. The hardware elements, operating systems, and programming languages of such computers are conventional in nature, and it is presumed that those skilled in the art are adequately familiar therewith to adapt those technologies to implement the processing essentially as described herein. A computer with user interface elements may be used to implement a personal computer (PC) or other type of work station or terminal device, although a computer may also act as a server if appropriately programmed. It is believed that those skilled in the art are familiar with the structure, programming, and general operation of such computer equipment and as a result the drawings should be self-explanatory.

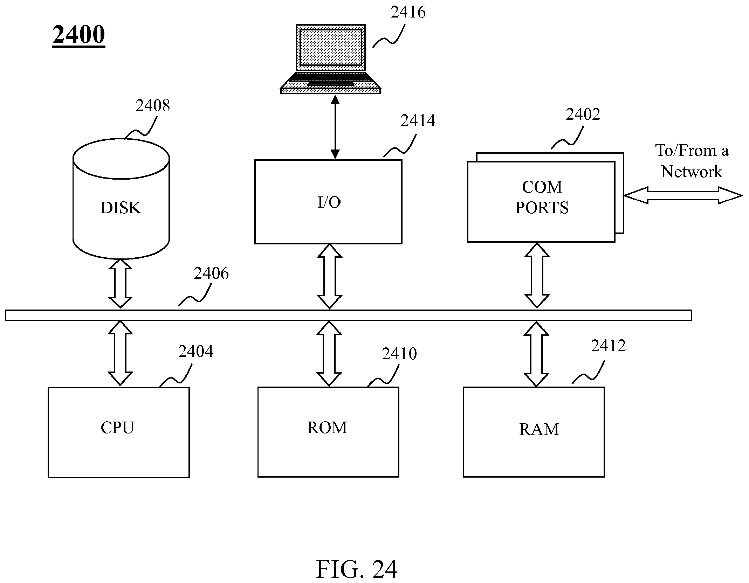

FIG. 24 depicts a general computer architecture on which the present teaching can be implemented and has a functional block diagram illustration of a computer hardware platform that includes user interface elements. The computer may be a general-purpose computer or a special purpose computer. This computer 2400 can be used to implement any components of the metal ion detection as described herein. Different components of the system, e.g., as depicted in FIGS. 4, 6 and 13, can all be implemented on one or more computers such as computer 2400, via its hardware, software program, firmware, or a combination thereof. Although only one such computer is shown, for convenience, the computer functions relating to metal ion detection may be implemented in a distributed fashion on a number of similar platforms, to distribute the processing load.

The computer 2400, for example, includes COM ports 2402 connected to and from a network connected thereto to facilitate data communications. The computer 2400 also includes a CPU 2404, in the form of one or more processors, for executing program instructions. The exemplary computer platform includes an internal communication bus 2406, program storage and data storage of different forms, e.g., disk 2408, read only memory (ROM) 2410, or random access memory (RAM) 2412, for various data files to be processed and/or communicated by the computer, as well as possibly program instructions to be executed by the CPU 2404. The computer 2400 also includes an I/O component 2414, supporting input/output flows between the computer and other components therein such as user interface elements 2416. The computer 2400 may also receive programming and data via network communications.

Hence, aspects of the method of metal ion detection, as outlined above, may be embodied in programming. Program aspects of the technology may be thought of as "products" or "articles of manufacture" typically in the form of executable code and/or associated data that is carried on or embodied in a type of machine readable medium. Tangible non-transitory "storage" type media include any or all of the memory or other storage for the computers, processors or the like, or associated modules thereof, such as various semiconductor memories, tape drives, disk drives and the like, which may provide storage at any time for the software programming.

All or portions of the software may at times be communicated through a network such as the Internet or various other telecommunication networks. Such communications, for example, may enable loading of the software from one computer or processor into another. Thus, another type of media that may bear the software elements includes optical, electrical, and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to tangible "storage" media, terms such as computer or machine "readable medium" refer to any medium that participates in providing instructions to a processor for execution.

Hence, a machine readable medium may take many forms, including but not limited to, a tangible storage medium, a carrier wave medium or physical transmission medium. Non-volatile storage media include, for example, optical or magnetic disks, such as any of the storage devices in any computer(s) or the like, which may be used to implement the system or any of its components as shown in the drawings. Volatile storage media include dynamic memory, such as a main memory of such a computer platform. Tangible transmission media include coaxial cables; copper wire and fiber optics, including the wires that form a bus within a computer system. Carrier-wave transmission media can take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a PROM and EPROM, a FLASH-EPROM, any other memory chip or cartridge, a carrier wave transporting data or instructions, cables or links transporting such a carrier wave, or any other medium from which a computer can read programming code and/or data. Many of these forms of computer readable media may be involved in carrying one or more sequences of one or more instructions to a processor for execution.

Those skilled in the art will recognize that the present teachings are amenable to a variety of modifications and/or enhancements. For example, although the implementation of various components described above may be embodied in a hardware device, it can also be implemented as a software only solution - e.g., an installation on an existing server. In addition, the units of the host and the client nodes as disclosed herein can be implemented as a firmware, firmware/software combination, firmware/hardware combination, or a hardware/firmware/software combination.

While the foregoing has described what are considered to be the best mode and/or other examples, it is understood that various modifications may be made therein and that the subject matter disclosed herein may be implemented in various forms and examples, and that the teachings may be applied in numerous applications, only some of which have been described herein.

## Claims

1. A method for detecting a level of a metal ion in a material, comprising:
detecting a first level of a magnetic field via a device comprising a magnetic sensor configured for sensing the magnetic field created by magnetic particles present in proximity to the magnetic sensor, wherein the device comprises a first set of breakable strands, wherein said breakable strands comprise functional nucleic acids having metal specificity, each of which having a first end previously immobilized with respect to the magnetic sensor and a second end attached to a magnetic particle;
depositing a sample of the material onto a base of the device that causes, due to the presence of the metal ion, at least some of the first set of breakable strands to break so that the magnetic particle attached to the second end of each of the at least some of the first set of breakable strands is no longer in proximity to the magnetic sensor;
detecting a second level of the magnetic field via the device; and
determining an amount of the metal ion present in the sample of the material based on the first and second levels of the magnetic field.

2. The method of claim 1, wherein the second level of the magnetic field is equal to or less than the first level of the magnetic field.

3. The method of claim 1, wherein:
the device further comprises a second set of strands;
each of the second set of strands is attached to a surface of the magnetic sensor;
and
the first end of each of the first set of breakable strands is bound to one of the second set of strands,
wherein preferably:
each of the first set of breakable strands comprises a first biological material; and
each of the second set of strands comprises a second biological material.

4. The method of claim 1, further comprising:
detecting, via the device comprising an additional magnetic sensor, a first level of an additional magnetic field created by magnetic particles present in proximity to the additional magnetic sensor, wherein:
the device comprises an additional set of strands each of which having a first end previously immobilized with respect to the additional magnetic sensor and a second end attached to a magnetic particle,
the sample of the material deposited onto the base comprises the metal ion and an additional substance, and
the additional substance causes at least some of the additional set of strands to break so that the magnetic particle attached to the second end of each of the at least some of the additional set of strands is no longer in proximity to the additional magnetic sensor;
detecting a second level of the additional magnetic field via the device; and
determining an amount of the additional substance present in the sample of the material based on the first and second levels of the additional magnetic field.

5. The method of claim 1, further comprising:
detecting, via the device comprising an additional magnetic sensor, a first level of an additional magnetic field created by magnetic particles present in proximity to the additional magnetic sensor, wherein:
the sample of the material deposited onto the base comprises the metal ion and
an additional substance, and
the additional substance causes at least some of an additional set of strands to be immobilized with respect to the additional magnetic sensor and causes some magnetic particles that are attached to the at least some of the additional set of strands to become in proximity to the additional magnetic sensor;
detecting a second level of the additional magnetic field via the device; and
determining an amount of the additional substance present in the sample of the material based on the first and second levels of the additional magnetic field.

6. A machine-readable tangible and non-transitory medium having information for detecting a level of a metal ion in a material, wherein the information, when read by a machine, causes the machine to perform the following:
detecting a first level of a magnetic field via a device comprising a magnetic sensor configured for sensing the magnetic field created by magnetic particles present in proximity to the magnetic sensor, wherein the device comprises a first set of breakable strands, wherein said breakable strands comprise functional nucleic acids having metal specificity, each of which having a first end previously immobilized with respect to the magnetic sensor and a second end attached to a magnetic particle;
depositing a sample of the material onto a base of the device that causes, due to the presence of the metal ion, at least some of the first set of breakable strands to break so that the magnetic particle attached to the second end of each of the at least some of the first set of breakable strands is no longer in proximity to the magnetic sensor;
detecting a second level of the magnetic field via the device; and
determining an amount of the metal ion present in the sample of the material based on the first and second levels of the magnetic field.

## Patentansprüche

1. Verfahren zum Detektieren eines Gehalts eines Metallions in einem Material, umfassend:
Detektieren einer ersten Stärke eines Magnetfelds durch eine Vorrichtung, umfassend einen Magnetsensor, der zum Erfassen des Magnetfelds, erzeugt durch in der Nähe zu dem Magnetsensor vorhandene magnetische Partikel, konfiguriert ist, wobei die Vorrichtung einen ersten Satz von brechbaren Strängen umfasst, wobei die brechbaren Stränge funktionelle Nukleinsäuren mit Metallspezifität umfassen, von denen jeder ein erstes Ende, das zuvor in Bezug auf den Magnetsensor immobilisiert wurde, und ein zweites Ende, das an ein magnetisches Partikel gebunden ist, umfasst;
Aufbringen einer Probe des Materials auf eine Basis der Vorrichtung, die bewirkt, dass aufgrund der Gegenwart des Metallions mindestens einige des ersten Satzes von brechbaren Strängen brechen, so dass das magnetische Partikel, das an das zweite Ende eines jeden der mindestens einigen des ersten Satzes von brechbaren Strängen gebunden ist, nicht länger in Nähe zu dem Magnetsensor ist;
Detektieren einer zweiten Stärke des Magnetfelds durch die Vorrichtung; und
Bestimmen eines Gehalts des Metallions, das in der Probe des Materials vorliegt, auf Basis der ersten und zweiten Stärke des Magnetfelds.

2. Verfahren nach Anspruch 1, wobei die zweite Stärke des Magnetfelds gleichstark oder weniger als die erste Stärke des Magnetfelds ist.

3. Verfahren nach Anspruch 1, wobei:
die Vorrichtung weiter einen zweiten Satz von Strängen umfasst; wobei jeder des zweiten Satzes von Strängen an eine Oberfläche des Magnetsensors gebunden ist; und
das erste Ende eines jeden des ersten Satzes von brechbaren Strängen an eines des zweiten Satzes von Strängen gebunden ist,
wobei bevorzugt:
jeder des ersten Satzes von brechbaren Strängen ein erstes biologisches Material umfasst; und
jeder des zweiten Satzes von Strängen ein zweites biologisches Material umfasst.

4. Verfahren nach Anspruch 1, weiter umfassend:
Detektieren durch die Vorrichtung, umfassend einen zusätzlichen Magnetsensor, einer erste Stärke eines zusätzlichen Magnetfelds, erzeugt durch in der Nähe zu dem zusätzlichen Magnetsensor vorhandene magnetische Partikel, wobei:
die Vorrichtung einen zusätzlichen Satz von Strängen, von denen jeder ein erstes Ende, das zuvor in Bezug auf den zusätzlichen Magnetsensor immobilisiert ist, und ein zweites Ende, das an ein magnetisches Partikel gebunden ist, umfasst, die auf der Basis aufgebrachte Probe des Materials das Metallion und eine zusätzliche Substanz umfasst, und
die zusätzliche Substanz bewirkt, dass mindestens einige des zusätzlichen Satzes von Strängen brechen, so dass das magnetische Partikel, das an das zweite Ende eines jeden der mindestens einigen des zusätzlichen Satzes von Strängen gebunden ist, nicht länger in Nähe zu dem zusätzlichen Magnetsensor ist;
Detektieren einer zweiten Stärke des zusätzlichen Magnetfelds durch die Vorrichtung; und
Bestimmen einer Menge der zusätzlichen Substanz, die in der Probe des Materials vorliegt, auf Basis der ersten und zweiten Stärken des zusätzlichen Magnetfelds.

5. Verfahren nach Anspruch 1, weiter umfassend:
Detektieren durch die Vorrichtung, umfassend einen zusätzlichen Magnetsensor, einer ersten Stärke eines zusätzlichen Magnetfelds, erzeugt durch in der Nähe zu dem zusätzlichen Magnetsensor vorhandene magnetische Partikel, wobei:
die auf der Basis aufgebrachte Probe des Materials das Metallion und eine zusätzliche Substanz umfasst, und
die zusätzliche Substanz bewirkt, dass mindestens einige eines zusätzlichen Satzes von Strängen in Bezug zu dem zusätzlichen Magnetsensor immobilisiert werden, und bewirkt, dass einige magnetische Partikel, die an die mindestens einige des zusätzlichen Satzes von Strängen gebunden sind, in Nähe zu dem zusätzlichen Magnetsensor gelangen;
Detektieren einer zweiten Stärke des zusätzlichen Magnetfelds durch die Vorrichtung; und
Bestimmen einer Menge der zusätzlichen Substanz, die in der Probe des Materials vorliegt, auf Basis der ersten und zweiten Stärken des zusätzlichen Magnetfelds.

6. Maschinenlesbares, greifbares und nicht-transitorisches Medium mit Informationen zum Detektieren eines Gehalts eines Metallions in einem Material, wobei die Information, wenn sie von einer Maschine gelesen wird, die Maschine veranlasst das Folgende durchzuführen:
Detektieren einer ersten Stärke eines Magnetfelds durch eine Vorrichtung, umfassend einen Magnetsensor, der zum Erfassen des Magnetfelds, erzeugt durch in der Nähe zu dem Magnetsensor vorhandene magnetische Partikel, konfiguriert ist, wobei die Vorrichtung einen ersten Satz von brechbaren Strängen umfasst, wobei die brechbaren Stränge funktionelle Nukleinsäuren mit Metallspezifität umfassen, von denen jede ein erstes Ende, das zuvor in Bezug auf den Magnetsensor immobilisiert ist, und ein zweites Ende, das an ein magnetisches Partikel gebunden ist, umfasst;
Aufbringen einer Probe des Materials auf eine Basis der Vorrichtung, die bewirkt, dass aufgrund der Gegenwart des Metallions mindestens einige des ersten Satzes von brechbaren Strängen brechen, so dass das magnetische Partikel, das an das zweite Ende eines jeden der mindestens einigen des ersten Satzes von brechbaren Strängen gebunden ist, nicht länger in Nähe zu dem Magnetsensor ist;
Detektieren einer zweiten Stärke des Magnetfelds durch die Vorrichtung; und Bestimmen einer Menge des Metallions, das in der Probe des Materials vorliegt, auf Basis der ersten und zweiten Stärken des Magnetfelds.

## Revendications

1. Méthode de détection d'un niveau d'un ion métal dans une matière, comprenant :
la détection d'un premier niveau d'un champ magnétique via un dispositif comprenant un détecteur magnétique configuré pour détecter le champ magnétique créé par des particules magnétiques présentes à proximité du détecteur magnétique, dans laquelle le dispositif comprend un premier jeu de brins séparables, dans laquelle lesdits brins séparables comprennent des acides nucléiques fonctionnels présentant une spécificité à des métaux, chacun d'eux ayant une première extrémité précédemment immobilisée par rapport au détecteur magnétique et une deuxième extrémité attachée à une particule magnétique ;
le dépôt d'un échantillon de la matière sur une base du dispositif qui fait qu'en présence de l'ion métal, au moins une partie du premier jeu de brins séparables se sépare de sorte que la particule magnétique attachée à la deuxième extrémité de chacun de la au moins une partie du premier jeu de brins séparables ne se trouve plus à proximité du détecteur magnétique ;
la détection du deuxième niveau de champ magnétique via le dispositif ; et
la détermination d'une quantité de l'ion métal présente dans l'échantillon de la matière sur base des premier et deuxième niveaux du champ magnétique.

2. Méthode selon la revendication 1, dans laquelle le deuxième niveau du champ magnétique est égal ou inférieur au premier niveau du champ magnétique.

3. Méthode selon la revendication 1, dans laquelle:
le dispositif comprend en outre un deuxième jeu de brins ;
chacun du deuxième jeu de brins est attaché à la surface du détecteur magnétique ;
et
la première extrémité de chacun du premier jeu de brins séparables est liée à l'un du deuxième jeu de brins,
dans laquelle, de préférence :
chacun du premier jeu de brins séparables comprend une première matière biologique ; et
chacun du deuxième jeu de brins comprend une deuxième matière biologique.

4. Méthode selon la revendication 1, comprenant en outre:
la détection, via le dispositif comprenant un détecteur magnétique supplémentaire, un premier niveau d'un champ magnétique supplémentaire créé par des particules magnétiques présentes à proximité du détecteur magnétique supplémentaire, dans laquelle
le dispositif comprend un jeu supplémentaire de brins, chacun d'eux ayant une première extrémité précédemment immobilisée par rapport au détecteur magnétique supplémentaire et une deuxième extrémité attachée à une particule magnétique, l'échantillon de la matière déposée sur la base comprend l'ion métal et une substance supplémentaire, et
la substance supplémentaire fait qu'au moins une partie du jeu supplémentaire de brins se sépare de sorte que la particule magnétique attachée à la deuxième extrémité de chacun de la au moins une partie du jeu supplémentaire de brins n'est plus à proximité du détecteur magnétique supplémentaire ;
la détection d'un deuxième niveau du champ magnétique supplémentaire via le dispositif ; et
la détermination d'une quantité de la substance supplémentaire présente dans l'échantillon de la matière sur base des premier et deuxième niveaux du champ magnétique supplémentaire.

5. Méthode selon la revendication 1, comprenant en outre:
la détection, via le dispositif comprenant un détecteur magnétique supplémentaire, d'un premier niveau d'un champ magnétique supplémentaire créé par des particules magnétiques présentes à proximité du détecteur magnétique supplémentaire, dans laquelle
l'échantillon de la matière déposée sur la base comprend l'ion métal et une substance supplémentaire, et
la substance supplémentaire fait qu'au moins une partie du jeu supplémentaire de brins soit immobilisée par rapport au détecteur magnétique supplémentaire et fait qu'une partie des particules magnétiques qui sont attachées à la au moins une partie du jeu supplémentaire de brins arrivent à proximité du détecteur magnétique supplémentaire ;
la détection d'un deuxième niveau du champ magnétique supplémentaire via le dispositif ; et
la détermination d'une quantité de la substance supplémentaire présente dans l'échantillon de la matière sur base des premier et deuxième niveaux du champ magnétique supplémentaire.

6. Support tangible et non transitoire pouvant être lu par une machine ayant des informations pour détecter le niveau d'un ion métal dans une matière, dans lequel les informations, lorsqu'elles sont lues par une machine, font que la machine réalise les suivants :
la détection d'un premier niveau d'un champ magnétique via un dispositif comprenant un détecteur magnétique configuré pour détecter le champ magnétique créé par des particules magnétiques présentes à proximité du détecteur magnétique, dans lequel le dispositif comprend un premier jeu de brins séparables, dans lequel lesdits brins séparables comprennent des acides nucléiques fonctionnels présentant une spécificité à des métaux, chacun d'eux ayant une première extrémité précédemment immobilisée par rapport au détecteur magnétique et une deuxième extrémité attachée à une particule magnétique ;
le dépôt d'un échantillon de la matière sur une base du dispositif qui fait qu'en présence de l'ion métal, au moins une partie du premier jeu de brins séparables se sépare de sorte que la particule magnétique attachée à la deuxième extrémité de chacun de la au moins une partie du premier jeu de brins séparables, ne se trouve plus à proximité du détecteur magnétique ;
la détection d'un deuxième niveau du champ magnétique via le dispositif ; et
la détermination d'une quantité de l'ion métal présente dans l'échantillon de la matière sur base des premier et deuxième niveaux du champ magnétique.
